# EUROPEAN PATENT APPLICATION

(11) **EP 4 364 750 A1**
(43) Date of publication of application: **08.05.2024**
(21) Application number: 22833674.9
(22) Date of filing: 30.06.2022
(51) Int. Cl.: A61K 38/16, A61K 38/26, A61K 47/68, A61K 47/60, A61K 31/7048, A61K 45/06, A61P 3/00, A61P 1/16, A61P 11/00, A61P 31/00

(54) **THERAPEUTIC USE OF COMBINATION COMPRISING TRIPLE ACTIVATOR HAVING ACTIVITY ON ALL OF GLUCAGON, GLP-1 AND GIP RECEPTOR**

(30) Priority: 30.06.2021 KR 20210086014
(71) Applicant: Hanmi Pharm. Co., Ltd., Hwaseong-si, Gyeonggi-do 18536 (KR)
(72) Inventor: LEE, Jong Suk, Hwaseong-si, Gyeonggi-do 18469 (KR); KIM, Yo Han, Hwaseong-si, Gyeonggi-do 18469 (KR); KIM, Jung Kuk, Hwaseong-si, Gyeonggi-do 18469 (KR); LEE, Sang Hyun, Hwaseong-si, Gyeonggi-do 18469 (KR)
(74) Representative: Mewburn Ellis LLP
(86) International application number: PCT/KR2022/009449
(87) International publication number: WO 2023/277620

(57) **Abstract**

The present invention relates to use of a combination including a peptide having activities to all of glucagon, GLP-1, and GIP receptors or a conjugate thereof; and an SGLT-2 inhibitor.

## Description

### [Technical Field]

The present invention relates to a combination including a peptide having activities to all of glucagon, GLP-1, and GIP receptors; or use of a combination including the peptide and an SGLT-2 inhibitor.

### [Background Art]

Glucagon is produced and secreted by the pancreas in response to low blood glucose levels due to various causes, such as drug treatment, diseases, hormone or enzyme deficiency, etc. It has been known that secreted glucagon acts on the liver to break down glycogen, thereby inducing the release of glucose and eventually raising blood glucose levels to normal levels. This glucagon exhibits activity by acting on glucagon receptor.

Further, glucagon-like peptide-1 (GLP-1) and glucose-dependent insulinotropic polypeptide (GIP), which are both representative gastrointestinal hormones and neuronal hormones, are known as substances involved in the control of blood glucose levels according to food intake.

Recently, there has arisen a need for a substance that can act on glucagon-like peptide-1 (GLP-1) and glucose-dependent insulinotropic polypeptide (GIP) receptors as well as glucagon receptor to increase efficacy or to reduce side effects, and therefore, the present inventors have developed peptides capable of acting on glucagon, GLP-1, and GIP receptors, and conjugates thereof (WO2017-116204; WO2017-116205).

Diabetes is a kind of metabolic disease in which insulin secretion is insufficient or normal functions are not made. Diabetes is characterized by hyperglycemia in which blood glucose levels are elevated, and is a disease which causes various conditions and syndromes due to hyperglycemia, and excretes glucose with urine.

One of the main causes of diabetes is overweight or obesity. Obesity is known to be a factor in the development of diabetes by causing increased insulin resistance and decreased insulin secretion. This relationship between diabetes and obesity is attributed to lipotoxicity resulting from accumulation of fatty acids in beta-cells or in insulin-sensitive tissues such as kidneys, liver, or heart due to irregular secretion of adipokines and free fatty acids.

In particular, obesity is a metabolic disease in itself, but it is known to cause diabetic complications in diabetic patients. Obesity is known not only to be a cause of diabetes, but also to be a factor that makes the prognosis of diabetic patients more serious. Diabetic patients with obesity have an increased risk of stroke, vascular complications, heart attack, diabetic retinopathy, renal dysfunction, chronic kidney disease, neuropathy, diabetic foot ulcers, and cardiovascular disease, and therefore, weight control is also required for effective treatment of diabetic patients.

Current main treatment method for diabetes relies on the control of blood glucose levels by drugs such as insulin, insulin secretion stimulators, insulin sensitivity enhancers, and blood glucose level lowering agents, etc.

Insulin, a common medication for diabetes, is prescribed when blood glucose control does not reach glycemic targets or when hyperglycemia is severe despite treatment with oral hypoglycemic agents. However, insulin is known to have side effects such as hypoglycemia, weight gain, etc.

Sodium-glucose cotransporter 2 (SGLT-2) inhibitors, which are a class of medicine used as blood glucose level lowering agents, are an oral diabetes treatment mainly used to control blood glucose in type 2 diabetes patients. It is known that SGLT-2 inhibitors inhibit SGLT-2, whose expression is increased in diabetic patients, thereby inhibiting glucose reabsorption by the kidneys and excreting glucose, resulting in a blood glucose lowering effect. In particular, since SGLT-2 inhibitors target glucose and act independently of insulin, they have the advantage of being able to be prescribed regardless of beta cell function or insulin resistance.

In addition, combination therapy, in which several drugs are administered together, is often used to treat diabetes with the aim of reducing insulin dependence, lowering the risk of hypoglycemia, and suppressing side effects such as weight gain.

Although the treatment of diabetes requires not only blood glucose control but also weight control, diabetic patients are required to consume an appropriate amount of food for treatment, which makes it difficult to lose weight through diet or exercise therapy, and the administration of anti-diabetic drugs, including insulin treatment, usually promotes adipogenesis during the insulin control process, causing obesity, or when blood glucose is controlled, the symptom of nutrient loss through urine is improved, leading to a phenomenon of weight gain.

Accordingly, if the effects of not only reducing blood glucose but also reducing body weight can be achieved in diabetic patients, it is expected to be an ultimate and effective treatment method for diabetes. In addition, since many obese patients also have diabetes, a therapy capable of achieving reduction in the body weight and blood glucose levels at the same time is also expected to be an effective treatment method for obesity.

Accordingly, as part of efforts to develop such treatment methods, efforts are ongoing to develop therapies to achieve the effects of blood glucose control and weight loss by administering two or more drugs in combination. However, as of now, there are no established therapeutic agents or therapies.

### [Disclosure]

### [Technical Problem]

There is a demand for the development of therapeutic agents or therapies capable of achieving blood glucose control and weight loss.

### [Technical Solution]

An object of the present invention is to provide a pharmaceutical composition for preventing or treating metabolic syndrome, liver disease, lung disease, or respiratory infections, the pharmaceutical composition including a peptide having activities to all of glucagon, GLP-1, and GIP receptors, or a conjugate thereof, wherein a sodium-glucose cotransporter 2 (SGLT-2) inhibitor is used in combination.

Another object of the present invention is to provide a combination including a peptide having activities to all of glucagon, GLP-1, and GIP receptors, or a conjugate thereof; and an SGLT-2 inhibitor.

Still another object of the present invention is to provide a pharmaceutical kit for preventing or treating metabolic syndrome, liver disease, lung disease, or respiratory infections, the pharmaceutical kit including a peptide having activities to all of glucagon, GLP-1, and GIP receptors, or a conjugate thereof; and an SGLT-2 inhibitor.

Still another object of the present invention is to provide a method of preventing or treating metabolic syndrome, liver disease, lung disease, or respiratory infections, the method including the step of administering a peptide having activities to all of glucagon, GLP-1, and GIP receptors, or a conjugate thereof; or a pharmaceutical composition including the same in combination with an SGLT-2 inhibitor, to a subject in need thereof.

Still another object of the present invention is to provide a method of preventing or treating metabolic syndrome, liver disease, lung disease, or respiratory infections, the method including the step of administering the combination or the pharmaceutical composition, and/or using the pharmaceutical kit to/in a subject in need thereof.

Still another object of the present invention is to provide use of the combination, the pharmaceutical composition, or the pharmaceutical kit in the prevention or treatment of metabolic syndrome, liver disease, lung disease, or respiratory infections, and/or use thereof in the preparation of a prophylactic or therapeutic agent for metabolic syndrome, liver disease, lung disease, or respiratory infections.

Still another object of the present invention is to provide a pharmaceutical composition for preventing or treating metabolic syndrome, liver disease, lung disease, or respiratory infections, the pharmaceutical composition including a peptide having activities to all of glucagon, GLP-1, and GIP receptors, or a conjugate thereof; and an SGLT-2 inhibitor.

### [Advantageous Effects]

Administration of a peptide having activities to all of glucagon, GLP-1, and GIP receptors of the present invention; or a conjugate thereof in combination with an SGLT-2 inhibitor may be used as an effective therapeutic regimen for the prevention or treatment of metabolic syndrome, liver disease, lung disease, or respiratory infections.

### [Brief Description of Drawings]

FIG. 1 shows the effects of reducing blood glucose and body weight by administering a long-acting conjugate of SEQ ID NO: 42 in combination with empagliflozin; and
FIG. 2 shows the results of area under curve (AUC) for analyzing the effects of reducing blood glucose by administering the long-acting conjugate of SEQ ID NO: 42 in combination with empagliflozin.

### [Best Mode for Carrying Out the Invention]

An aspect of the present invention provides a composition including a peptide having activities to all of glucagon, GLP-1, and GIP receptors, or a conjugate thereof.

With regard to the composition according to a specific embodiment, the composition is a pharmaceutical composition for preventing or treating metabolic syndrome, liver disease, lung disease, or respiratory infections, the pharmaceutical composition including a pharmaceutically effective amount of the peptide having activities to all of glucagon, GLP-1, and GIP receptors; and a pharmaceutically acceptable excipient, wherein the pharmaceutical composition is used in combination with a sodium-glucose cotransporter 2 (SGLT-2) inhibitor.

With regard to the composition according to another specific embodiment, the peptide having activities to all of glucagon, GLP-1, and GIP receptors is a peptide including any one amino acid sequence of SEQ ID NOS: 1 to 102.

With regard to the composition according to any one of the previous specific embodiments, the peptide is in the form of a long-acting conjugate, wherein the long-acting conjugate is represented by Formula 1 below:

[Formula 1] X-L-F

wherein X represents a peptide including any one amino acid sequence of SEQ ID NOS: 1 to 102;
L represents a linker containing ethylene glycol repeating units;
F represents an immunoglobulin Fc region; and
   - represents covalent linkages between X and L and between L and F, respectively.

With regard to the composition according to any one of the previous specific embodiments, the metabolic syndrome is one or more selected from the group consisting of diabetes, obesity, hyperlipidemia, and dyslipidemia.

With regard to the composition according to any one of the previous specific embodiments, the SGLT-2 inhibitor is one or more selected from the group consisting of empagliflozin, dapagliflozin, canagliflozin, remogliflozin, remogliflozin etabonate, sergliflozin, ipragliflozin, tofogliflozin, luseogliflozin, sotagliflozin, bexagliflozin, atigliflozin, and ertugliflozin.

With regard to the composition according to any one of the previous specific embodiments, the composition has weight loss and/or blood glucose lowering effects.

With regard to the composition according to any one of the previous specific embodiments, the composition has the blood glucose lowering effect in diabetic patients with overweight or obesity.

With regard to the composition according to any one of the previous specific embodiments, the composition has the blood glucose lowering effect in obese patients with hyperglycemia or diabetes.

With regard to the composition according to any one of the previous specific embodiments, the peptide includes any one amino acid sequence of SEQ ID NOS: 21, 22, 42, 43, 50, 77, and 96.

With regard to the composition according to any one of the previous specific embodiments, the peptide is not C-terminally modified or the peptide is C-terminally amidated.

With regard to the composition according to any one of the previous specific embodiments, the peptide has a ring formed between amino acid residues.

With regard to the composition according to any one of the previous specific embodiments, the immunoglobulin Fc region is aglycosylated.

With regard to the composition according to any one of the previous specific embodiments, the immunoglobulin Fc region is selected from the group consisting of (a) a CH1 domain, a CH2 domain, a CH3 domain, and a CH4 domain; (b) a CH1 domain and a CH2 domain; (c) a CH1 domain and a CH3 domain; (d) a CH2 domain and a CH3 domain; (e) a combination of one domain or two or more domains of a CH1 domain, a CH2 domain, a CH3 domain, and a CH4 domain, and an immunoglobulin hinge region or a part of the hinge region; and (f) a dimer of each domain of a heavy chain constant region and a light chain constant region.

With regard to the composition according to any one of the previous specific embodiments, the immunoglobulin Fc region is a native Fc in which a site capable of forming a disulfide bond is deleted, a native Fc in which some amino acids at the N-terminus are deleted, or a native Fc in which a methionine residue is added at the N-terminus, a complement-binding site is deleted, or an antibody-dependent cell-mediated cytotoxicity (ADCC) site is deleted.

With regard to the composition according to any one of the previous specific embodiments, the immunoglobulin Fc region is derived from IgG, IgA, IgD, IgE or IgM.

With regard to the composition according to any one of the previous specific embodiments, the immunoglobulin Fc region is a hybrid of domains with different origins derived from immunoglobulins selected from the group consisting of IgG, IgA, IgD, IgE, and IgM.

With regard to the composition according to any one of the previous specific embodiments, the immunoglobulin Fc region has a dimeric form.

With regard to the composition according to any one of the previous specific embodiments, the immunoglobulin Fc region is a dimer consisting of two polypeptide chains, wherein one end of L is linked to only one polypeptide chain of the two polypeptide chains.

With regard to the composition according to any one of the previous specific embodiments, the immunoglobulin Fc region is an IgG4 Fc region.

With regard to the composition according to any one of the previous specific embodiments, the immunoglobulin Fc region is an aglycosylated Fc region derived from human IgG4.

With regard to the composition according to any one of the previous specific embodiments, in the conjugate, L is linked to F and X by covalent linkages formed by reacting one end of L with an amine group or thiol group of F and reacting the other end of L with an amine group or thiol group of X, respectively.

With regard to the composition according to any one of the previous specific embodiments, L is polyethylene glycol.

With regard to the composition according to any one of the previous specific embodiments, a formula weight of a moiety of the ethylene glycol repeating units in L is in the range of 1 kDa to 100 kDa.

With regard to the composition according to any one of the previous specific embodiments, the composition has the weight loss effect in a subject with obesity.

With regard to the composition according to any one of the previous specific embodiments, the ethylene glycol repeating units are [OCH₂CH₂]ₙ, wherein n is a natural number, and is determined such that an average molecular weight, for example, a number average molecular weight of the [OCH₂CH₂]ₙ moiety in the peptide conjugate is 1 kDa to 100 kDa.

With regard to the composition according to any one of the previous specific embodiments, the value of n is determined such that the average molecular weight, for example, the number average molecular weight, of the [OCH₂CH₂]ₙ moiety in the peptide conjugate is 10 kDa.

Another aspect of the present invention provides a combination including a peptide having activities to all of glucagon, GLP-1, and GIP receptors, or a conjugate thereof; and an SGLT-2 inhibitor.

Still another aspect of the present invention provides a pharmaceutical kit for preventing or treating metabolic syndrome, liver disease, lung disease, or respiratory infections, the pharmaceutical kit including a peptide having activities to all of glucagon, GLP-1, and GIP receptors, or a conjugate thereof; and an SGLT-2 inhibitor.

With regard to the pharmaceutical kit of a specific embodiment, the metabolic syndrome is any one or more selected from the group consisting of diabetes, obesity, hyperlipidemia, and dyslipidemia.

Still another aspect of the present invention provides a method of preventing or treating metabolic syndrome, liver disease, lung disease, or respiratory infections, the method including the step of administering and/or using the combination, pharmaceutical composition, or the pharmaceutical kit to/in a subject in need thereof.

With regard to the method of a specific embodiment, the metabolic syndrome is any one or more selected from the group consisting of diabetes, obesity, hyperlipidemia, and dyslipidemia.

Still another aspect of the present invention provides a method of preventing or treating metabolic syndrome, liver disease, lung disease, or respiratory infections, the method including the step of administering a peptide having activities to all of glucagon, GLP-1, and GIP receptors, or a conjugate thereof; or a pharmaceutical composition including the same in combination with an SGLT-2 inhibitor, to a subject in need thereof.

With regard to the method of a specific embodiment, the metabolic syndrome is any one or more selected from the group consisting of diabetes, obesity, hyperlipidemia, and dyslipidemia.

Still another aspect of the present invention provides use of the combination, the pharmaceutical composition, or the pharmaceutical kit in the prevention or treatment of metabolic syndrome, liver disease, lung disease, or respiratory infections, and/or use thereof in the preparation of a prophylactic or therapeutic agent for metabolic syndrome, liver disease, lung disease, or respiratory infections.

With regard to the use of a specific embodiment, the metabolic syndrome is any one or more selected from the group consisting of diabetes, obesity, hyperlipidemia, and dyslipidemia.

Still another aspect of the present invention provides a pharmaceutical composition for preventing or treating metabolic syndrome, liver disease, lung disease, or respiratory infections, the pharmaceutical composition including a peptide having activities to all of glucagon, GLP-1, and GIP receptors, or a conjugate thereof; and an SGLT-2 inhibitor.

With regard to the composition of a specific embodiment, the metabolic syndrome is any one or more selected from the group consisting of diabetes, obesity, hyperlipidemia, and dyslipidemia.

### [Mode for Carrying Out the Invention]

Hereinafter, the present invention will be described in more detail.

Meanwhile, each description and embodiment disclosed in this disclosure may also be applied to other descriptions and embodiments. That is, all combinations of various elements disclosed in this disclosure fall within the scope of the present invention. Further, the scope of the present invention is not limited by the specific description described below.

Throughout the description, not only the typical one-letter and three-letter codes for naturally occurring amino acids, but also three-letter codes generally allowed for other amino acids, such as 2-aminoisobutyric acid (Aib), N-methylglycine (Sar), and α-methyl-glutamic acid, are used. Further, the amino acids mentioned in abbreviations herein are described according to the IUPAC-IUB rules.

| | |
|---|---|
| alanine Ala, A | arginine Arg, R |
| asparagine Asn, N | aspartic acid Asp, D |
| cysteine Cys, C | glutamic acid Glu, E |
| glutamine Gln, Q | glycine Gly, G |
| histidine His, H | isoleucine Ile, I |
| leucine Leu, L | lysine Lys, K |
| methionine Met, M | phenylalanine Phe, F |
| proline Pro, P | serine Ser, S |
| threonine Thr, T | tryptophan Trp, W |
| tyrosine Tyr, Y | valine Val, V |

As used herein, "Aib" may be used interchangeably with "2-aminoisobutyric acid" or "aminoisobutyric acid", and 2-aminoisobutyric acid and aminoisobutyric acid may be used interchangeably with each other.

In accordance with an aspect of the present invention, there is provided a composition including a peptide having activities to glucagon receptor, glucagon-like peptide-1 (GLP-1) receptor, and glucose-dependent insulinotropic polypeptide (GIP) receptor, or a conjugate thereof. Specifically, the composition is a pharmaceutical composition for preventing or treating metabolic syndrome, liver disease, lung disease, or respiratory infections, the pharmaceutical composition including the peptide or the conjugate thereof. Specific examples of the metabolic syndrome may include diabetes, obesity, hyperlipidemia, and dyslipidemia, but are not limited thereto.

According to a specific aspect of the present invention, there is provided a composition including a peptide having activities to glucagon receptor, glucagon-like peptide-1 (GLP-1) receptor, and glucose-dependent insulinotropic polypeptide (GIP) receptor, or a conjugate thereof, wherein the composition is a pharmaceutical composition for preventing or treating diabetes and/or obesity.

In an embodiment, the peptide may include, may essentially consist of, or may consist of any one amino acid sequence of SEQ ID NOS: 1 to 102.

In another embodiment, the composition may be a pharmaceutical composition including a pharmaceutically acceptable excipient and a peptide including any one amino acid sequence of SEQ ID NOS: 1 to 102 or a conjugate thereof in a pharmaceutically effective amount.

The composition including the peptide according to the present invention or a conjugate thereof as an active ingredient may be used in combination with a sodium-glucose cotransporter (SGLT-2) inhibitor.

Specifically, since the composition of the present invention may exhibit not only the blood glucose lowering effect but also the weight loss effect by being administered in combination with the SGLT-2 inhibitor, it may be effective treatment for patients with metabolic syndrome who require both blood glucose control and weight control, such as diabetic patients (e.g., diabetic patients with overweight or obesity), or obese patients (e.g., obese patients with hyperglycemia or diabetes).

Further, since the peptide of the present invention or the conjugate thereof; or the composition including the same has anti-inflammatory and/or anti-fibrotic effects, it may also be effective in liver disease, lung disease, or respiratory infections by being administered in combination with the SGLT-2 inhibitor.

As used herein, "combination administration", "used in combination", or "administered in combination" means that the peptide according to the present invention or the conjugate thereof; or the composition including the same as an active ingredient is administered along with the SGLT-2 inhibitor to a subject. This not only means simultaneous administration of the drugs to be used in combination, but also should be understood as a dosage form that enables each substance to perform its function at a level equivalent to or higher than its original function by acting on a subject the peptide having activities to glucagon receptor, GLP-1 receptor, and GIP receptor or the conjugate thereof, together with the SGLT-2 inhibitor. Therefore, when the term "in combination" is used herein, it refers to simultaneous, separate, sequential, or reverse sequential administration, and it should be understood that the order is not limited. When the administration is sequential, reverse sequential, or separate administration, the order of administration is not particularly limited. However, the interval of the secondary ingredient administration should be such that the beneficial effects of the combination administration are not lost.
(i) The peptide having activities to glucagon receptor, GLP-1 receptor, and GIP receptor, or the conjugate thereof; and (ii) the SGLT-2 inhibitor which are administered in combination in the present invention may be administered in the following form, but are not limited thereto:
   a) administered in a single mixture, in which (i) the peptide having activities to glucagon receptor, GLP-1 receptor, and GIP receptor, or the conjugate thereof; and (ii) the SGLT-2 inhibitor are mixed; or
   b) administered in a separate form, in which (i) the peptide having activities to glucagon receptor, GLP-1 receptor, and GIP receptor, or the conjugate thereof; and (ii) the SGLT-2 inhibitor are separated, but are not limited thereto.

When the peptide having activities to glucagon receptor, GLP-1 receptor, and GIP receptor, or the conjugate thereof, and the SGLT-2 inhibitor are in a separate form, the substance having activities to glucagon receptor, GLP-1 receptor, and GIP receptor, or the conjugate thereof, and the SGLT-2 inhibitor are formulated into separate preparations, which may be administered simultaneously, separately, sequentially, or reverse sequentially.

The present invention may obtain the blood glucose lowering effect and the weight loss effect at the same time when the SGLT-2 inhibitor is further included and used in combination with the peptide having activities to glucagon receptor, GLP-1 receptor, and GIP receptor, or the conjugate thereof, and therefore, it was confirmed that the effect of preventing or treating diabetes and/or obesity is dramatically improved, thereby providing the above combination therapy.

Further, when the SGLT-2 inhibitor is further included and used in combination with the peptide having activities to glucagon receptor, GLP-1 receptor, and GIP receptor, or the conjugate thereof according to the present invention, the effect of preventing or treating metabolic syndrome, liver disease, lung disease, or respiratory infections may be obtained.

As used herein, the blood glucose lowering means lowering blood glucose levels. Blood glucose in the body maintains homeostasis by insulin and glucagon. When blood glucose exceeds the normal range to be maintained at high levels, diabetes occurs. The SGLT-2 inhibitor, which is an oral therapeutic agent for diabetes, functions to promote the excretion of glucose and to lower blood glucose. When the peptide according to the present invention or the conjugate thereof is administered in combination with the SGLT-2 inhibitor, it is possible to lower blood glucose as well as to reduce a patient's weight, thereby treating diabetes along with obesity, which is a cause of diabetes, and preventing complications of diabetes. From this point of view, it may be an advantageous therapy. In addition, the combination administration may be an effective treatment option for obese patients with hyperglycemia or diabetes because it exhibits the blood glucose lowering effect and the weight loss effect at the same time.

The "peptide having activities to glucagon receptor, GLP-1 receptor, and GIP receptor" of the present invention may also be used interchangeably with the term "triple agonist" or "peptide" in the present invention.

Such a peptide may include various substances, for example, various peptides, which have significant levels of activities to glucagon, GLP-1, and GIP receptors.

Although not particularly limited, the peptide having significant levels of activities to glucagon, GLP-1, and GIP receptors may exhibit *in vitro* activities to one or more receptors, specifically two or more receptors, and more specifically all three of the receptors among glucagon, GLP-1, and GIP receptors, of about 0.001 %or more, about 0.01% or more, about 0.1% or more, about 1% or more, about 2% or more, about 3 % or more, about 4% or more, about 5% or more, about 6% or more, about 7% or more, about 8% or more, about 9% or more, about 10 % or more, about 20% or more, about 30% or more, about 40% or more, about 50 % or more, about 60% or more, about 70% or more, about 80% or more, about 90% or more, about 100% or more, about 150% or more, about 200% or more, as compared with native ligands for the corresponding receptors (native glucagon, native GLP-1, and native GIP), but any range with a significant increase is included without limitation.

Here, the activities to the receptors may be exemplified by those cases where the *in vitro* activities to the receptors are about 0.001 % or more, 0.01% or more, 0.1% or more, 1% or more, 2% or more, 3% or more, 4% or more, 5% or more, 6% or more, 7% or more, 8% or more, 9% or more, 10% or more, 20% or more, 30% or more, 40% or more, 50% or more, 60% or more, 70% or more, 80% or more, 90% or more, 100% or more, about 200% or more, as compared with the native forms, but are not limited thereto.

As used herein, the term "about" refers to a range including ±0.5, ±0.4, ±0.3, ±0.2, ±0.1, etc., and thus includes all of the values in the range equivalent or similar to those stated after the term "about", but is not limited thereto.

Reference is made to Experimental Example 1 herein for methods of measuring the *in vitro* activities of such triple agonists, but is not particularly limited thereto.

Meanwhile, the peptide is characterized by retaining one or more, two or more, specifically, three of the following activities of i) to iii) below, specifically significant activities thereof:
i) activation of GLP-1 receptor; ii) activation of glucagon receptor; and iii) activation of GIP receptor.

Here, the activation of the receptors may be exemplified by those cases where the *in vitro* activities to the receptors are about 0.001% or more, about 0.01% or more, about 0.1% or more, about 1% or more, about 2% or more, about 3% or more, about 4% or more, about 5% or more, about 6% or more, about 7% or more, about 8% or more, about 9% or more, about 10% or more, about 20% or more, about 30% or more, about 40% or more, about 50% or more, about 60% or more, about 70% or more, about 80% or more, about 90% or more, about 100% or more, about 150% or more, about 200% or more, as compared with native forms, but is not limited thereto.

Further, the peptide may have an increased *in vivo* half-life, as compared with any one of native GLP-1, native glucagon, and native GIP, but is not particularly limited thereto.

For example, the peptide of the present invention may also include a peptide that includes any one amino acid sequence of SEQ ID NOS: 1 to 102, or (essentially) consists of any one amino acid sequence of SEQ ID NOS: 1 to 102, or has at least 60% or more, 65% or more, 70% or more, 75% or more, 80% or more, 85% or more, 90% or more, 91 % or more, 92% or more, 93% or more, 94% or more, or 95% or more sequence identity to any one amino acid sequence of SEQ ID NOS: 1 to 102, and with respect to the objects of the present invention, as long as the peptide has the blood glucose lowering and weight loss effects when administered in combination with the SGLT-2 inhibitor, it is not limited to a particular sequence.

Much more specifically, the peptide may include or may (essentially) consist of any one amino acid sequence of SEQ ID NOS: 21, 22, 42, 43, 50, 77, and 96, but is not limited thereto.

Although described as a peptide "consisting of" a specific SEQ ID NO herein, such description does not exclude a mutation that may occur by the addition of a meaningless sequence upstream or downstream of the amino acid sequence of the corresponding SEQ ID NO, or a mutation that may occur naturally, or a silent mutation thereof, as long as the peptide has an activity identical or corresponding to that of the peptide consisting of the amino acid sequence of the corresponding SEQ ID NO, and it would be obvious that even a peptide with such a sequence addition or mutation falls within the scope of the present invention. In other words, when a peptide exhibits a predetermined level of homology and shows activity for glucagon receptor, GLP-1 receptor, and/or GIP receptor despite having some sequence differences, such a peptide may fall within the scope of the present invention.

For example, reference may be made to WO2017-116204 and WO2017-116205 for the peptide of the present invention.

As used herein, the term 'homology' or 'identity' refers to a degree of relatedness between two given amino acid sequences or nucleotide sequences, and may be expressed as a percentage.

The terms 'homology and identity' may be often used interchangeably.

Whether or not any two peptide sequences have homology, similarity, or identity may be determined by, for example, a known computer algorithm, such as the "FASTA" program, by using default parameters as in Pearson et al (1988)[Proc. Natl. Acad. Sci. USA 85]: 2444. Alternatively, it may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, the homology, similarity, or identity may be determined using BLAST or ClustalW of the National Center for Biotechnology Information Database.

The homology, similarity, or identity of peptides may be determined by comparing sequence information using a GAP computer program, e.g., Needleman et al. (1970), J Mol Biol.48: 443, as disclosed in Smith and Waterman, Adv. Appl. Math (1981) 2:482. Briefly, the GAP program defines similarity as the number of aligned symbols (i.e., amino acids) which are similar, divided by the total number of symbols in the shorter of the two sequences. The default parameters for the GAP program may include: (1) a unary comparison matrix (containing a value of 1 for identities and 0 for non-identities) and the weighted comparison matrix (or EDNAFULL (EMBOSS version of NCBI NUC4.4) substitution matrix) of Gribskov et al(1986) Nucl. Acids Res. 14: 6745, as disclosed by Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap open penalty 10, gap extension penalty 0.5); and (3) no penalty for end gaps. Therefore, the term "homology" or "identity" used herein represents the relevance between sequences.

The peptide of the present invention may include an intramolecular bridge (e.g., a covalent bridge or non-covalent bridge), and specifically, may be in the form of including a ring, for example, may be in the form of including a ring formed between the amino acid at position 16 and the amino acid at position 20 of the peptide, but the peptide is not particularly limited thereto. For specific example, the amino acid at position 16 may be glutamic acid, and the amino acid at position 20 may be lysine, but are not limited thereto.

Non-limiting examples of the ring may include a lactam bridge (or a lactam ring).

Further, the peptide includes all of those which are modified to include a ring, or to include amino acids capable of forming a ring at a target position.

For example, the pair of the amino acids at positions 16 and 20 in the peptide may be substituted with glutamic acid or lysine, which is able to form a ring, but is not limited thereto.

Such a ring may be formed between amino acid side chains in the peptide, for example, in the form of a lactam ring formed between a side chain of lysine and a side chain of glutamic acid, but is not particularly limited thereto.

Examples of the peptide prepared by a combination of these methods include a peptide, of which the amino acid sequence differs from that of native glucagon in one or more amino acids, from which the α-carbon of the amino acid residue at the N-terminus is removed, and which retains activities to glucagon receptor, GLP-1 receptor, and GIP receptor, but are not limited thereto. Peptides applicable to the present invention may be prepared by combining various methods for the preparation of analogs.

Further, in the peptide of the present invention, some amino acids may be substituted with other amino acids or non-natural compounds to avoid the recognition by an agonist protease, for increasing the *in vivo* half-life of the peptide, but the peptide is not particularly limited thereto.

Specifically, the peptide may be a peptide in which the *in vivo* half-life is increased by avoiding the recognition by a degradation enzyme through a substitution of the second amino acid in the amino acid sequence of the peptide, but any substitution or modification of amino acids to avoid recognition by an *in vivo* degradation enzyme is included without limitation.

Such a modification for peptide preparation includes all of: modifications using L-type or D-type amino acids and/or non-native amino acids; and/or modifications of native sequence, for example, a variation of a side chain functional group, an intramolecular covalent linkage, e.g., ring formation between side chains, methylation, acylation, ubiquitination, phosphorylation, aminohexanation, biotinylation, or the like.

Further, all of those in which one or more amino acids are added to the amino and/or carboxy terminus are included.

The substituted or added amino acids may be not only 20 amino acids that are commonly found in human proteins, but also atypical amino acids or non-naturally occurring amino acids. Commercial sources of atypical amino acids may include Sigma-Aldrich, ChemPep, and Genzyme Pharmaceuticals. The peptides including these amino acids and typical peptide sequences may be synthesized and purchased from commercial peptide suppliers, for example, American Peptide Company and Bachem in the USA, or Antigen in Korea.

Amino acid derivatives may also be accessible in a similar manner, and for example, 4-imidazoacetic acid or the like may be used.

The peptide according to the present invention may be in a modified form in which the N-terminus and/or C-terminus is chemically modified or protected by organic groups, or amino acids are added to the terminus of the peptide, for its protection from proteases *in vivo* while increasing its stability.

In particular, a chemically-synthesized peptide has electrically charged N- and C-termini, and thus for elimination of these charges, the N-terminus may be acetylated and/or the C-terminus may be amidated, but the peptide is not particularly limited thereto.

Specifically, the N-terminus or the C-terminus of the peptide of the present invention may have an amine group (-NH₂) or a carboxy group (-COOH), but is not limited thereto.

Further, the peptide of the present invention may be synthesized depending on the length thereof by a method well known in the art, for example, an automatic peptide synthesizer, and may be produced by genetic engineering technology.

Specifically, the peptide of the present invention may be prepared by a standard synthesis method, a recombinant expression system, or any other method known in the art. Therefore, the peptide according to the present invention may be synthesized by a number of methods including, for example, the following methods:
(a) a method of synthesizing a peptide by means of solid phase or liquid phase methodology either stepwise or by fragment assembling, followed by isolation and purification of a final peptide product;
(b) a method of expressing a nucleic acid construct encoding a peptide in a host cell and recovering an expression product from the host cell culture;
(c) a method of performing an *in vitro* cell-free expression of a nucleic acid construct encoding a peptide and recovering an expression product therefrom; or
a method of obtaining peptide fragments by any combination of the methods (a), (b), and (c), obtaining the peptide by linking the fragments, and then recovering the peptide.

Further, the peptide having activities to glucagon receptor, GLP-1 receptor, and GIP receptor may be in the form of a long-acting conjugate, in which a biocompatible material for increasing the *in vivo* half-life of the peptide having activities to glucagon receptor, GLP-1 receptor, and GIP receptor is conjugated to the peptide. Herein, the biocompatible material may be used interchangeably with a carrier.

In the present invention, a conjugate of the peptide may exhibit an increased duration of efficacy, as compared with the peptide to which a carrier is not conjugated, and in the present invention, such a conjugate is referred to as a "long-acting conjugate".

As used herein, the term "long-acting conjugate" or "conjugate" has a structure in which a biocompatible material is conjugated to the peptide, and may exhibit an increased duration of efficacy, as compared with the peptide to which the biocompatible material is not conjugated. In the long-acting conjugate, the biocompatible material may be linked to the peptide by a covalent linkage, but is not particularly limited thereto. In the present invention, the peptide, which is one element of the conjugate, may be a peptide having activities to glucagon receptor, GLP-1 receptor, and GIP receptor, and specifically, a peptide or fragment including any one amino acid sequence of the amino acid sequences of SEQ ID NO: 1 to 102, and the biocompatible material is a substance capable of increasing the half-life of the peptide and corresponds to one element of a moiety constituting the conjugate of the present invention.

For example, the peptide included in the conjugate may be a peptide including or (essentially) consisting of any one amino acid sequence of the amino acid sequences of SEQ ID NO: 1 to 102. Further, the peptide may have the increased half-life without losing activity by binding to the biocompatible material. However, as long as the peptide has the blood glucose lowering and weight loss effects when administered in combination with the SGLT-2 inhibitor, it may be used as one element constituting the conjugate of the present invention without limitation.

As used herein, the term "biocompatible material" refers to a substance that may be conjugated to the peptide of the present invention which is a physiologically active substance, thereby increasing the duration of efficacy of the physiologically active substance, as compared with a physiologically active substance to which a biocompatible material moiety or carrier is not conjugated. The biocompatible material may be covalently linked to a physiologically active substance (e.g., peptide), but is not particularly limited thereto.

The biocompatible material may be an immunoglobulin Fc region, more specifically, an IgG Fc region, but is not particularly limited thereto.

One or more amino acid side chains within the peptide of the present invention may be joined to such a biocompatible material to increase solubility and/or half-life *in vivo,* and/or to increase bio-availability thereof. Such a modification may also reduce the clearance of therapeutic proteins and peptides.

The biocompatible materials described above may be water-soluble (amphipathic or hydrophilic) and/or nontoxic and/or pharmaceutically acceptable.

Meanwhile, these conjugates may be non-naturally occurring.

In a specific embodiment of the present invention, the long-acting conjugate or conjugate may be represented by Formula 1 below, but is not limited thereto:

[Formula 1] X-L-F

wherein X represents a peptide including any one amino sequence of SEQ ID NOS: 1 to 102;
L represents a linker containing ethylene glycol repeating units;
F represents an immunoglobulin Fc region or a derivative thereof; and
   - represents covalent linkages between X and L and between L and F, respectively.

The conjugate of the present invention, even in the form of a conjugate, may exhibit significant activities to glucagon receptor, GLP-1 receptor, and GIP receptor, and thus may also exert the blood glucose lowering and weight loss effects when administered in combination with the SGLT-2 inhibitor.

Specifically, the conjugate of the present invention may exhibit *in vitro* activities to glucagon receptor, GLP-1 receptor, and/or GIP receptor, of about 0.001% or more, 0.01% or more, 0.1% or more, 0.1% or more, 0.2% or more, 0.5% or more, 0.7% or more, 1% or more, 2% or more, 3% or more, 4% or more, 5% or more, 6% or more, 7% or more, 8% or more, 9% or more, 10% or more, 20% or more, 30% or more, 40% or more, 50% or more, 60% or more, 70% or more, 80% or more, 90% or more, 100% or more, as compared with native forms, but is not limited thereto.

With respect to the objects of the present invention, the peptide or the conjugate thereof may exhibit activities to glucagon receptor, GLP-1 receptor, and/or GIP receptor, of about 0.001 % or more, 0.01% or more, 0.1% or more, 1% or more, 2% or more, 3% or more, 4% or more, 5% or more, 10% or more, 20% or more, 30% or more, 40% or more, 50% or more, 60% or more, 70% or more, 80% or more, 90% or more, 100% or more, as compared with native forms, but is not limited thereto.

In the conjugate, X may include any one amino acid sequence of the amino acid sequences of SEQ ID NOS: 1 to 102, or may essentially consist of or may consist of any one amino acid sequence of the amino acid sequences of SEQ ID NOS: 1 to 102, specifically, X may include any one amino acid sequence of SEQ ID NOS: 21, 22, 42, 43, 50, 77, and 96, or may essentially consist of or may consist of any one amino acid sequence of SEQ ID NOS: 21, 22, 42, 43, 50, 77, and 96, but is not limited thereto.

In the conjugate, F is a substance capable of increasing the half-life of X, i.e., a peptide having activities to glucagon receptor, GLP-1 receptor, and GIP receptor, specifically, a peptide including any one sequence of the amino acid sequences of SEQ ID NOS: 1 to 102, and corresponds to one element of a moiety constituting the conjugate of the present invention.

F and X may be linked to each other by a covalent chemical linkage or a non-covalent chemical linkage, and F and X may be linked to each other via L by a covalent chemical linkage, a non-covalent chemical linkage, or a combination thereof.

More specifically, X and L, and L and F may be linked to each other by a covalent linkage, wherein in the conjugate, X, L, and F are linked to each other through a covalent linkage in the order shown in Formula 1.

F may be an immunoglobulin Fc region, more specifically, the immunoglobulin Fc region may be derived from IgG, but is not particularly limited thereto.

As used herein, the term "immunoglobulin Fc region" refers to a region that includes heavy chain constant region 2(CH2) and/or heavy chain constant region 3(CH3), excluding heavy chain and light chain variable regions of an immunoglobulin. The immunoglobulin Fc region may be one element constituting a moiety of the conjugate of the present invention.

In the present invention, an Fc region includes not only the native sequence obtained by papain digestion of immunoglobulins, but also derivatives thereof, for example, variants in which one or more amino acid residues in the native sequence are modified by deletion, insertion, non-conservative or conservative substitution, or a combination thereof and is thus different from that of the native form.

F has a structure in which two polypeptide chains are linked by a disulfide bond, wherein the two polypeptide chains are linked through only a nitrogen atom of one chain of the two chains, but is not limited thereto. The linking through a nitrogen atom may be linking to the epsilon amino atoms of lysine or the N-terminus amino group through reductive amination.

The reductive amination refers to a reaction in which an amine group or an amino group of one reactant reacts with an aldehyde (i.e., a functional group capable of reductive amination) of another reactant to form an amine, and then an amine linkage is formed by reduction, and the reductive amination is an organic synthetic reaction widely known in the art.

In a specific embodiment, F may be linked through a nitrogen atom of the N-terminus proline thereof, but is not limited thereto.

The immunoglobulin Fc region may be one element constituting a moiety of the conjugate of Formula 1 of the present invention, and specifically, may correspond to F in Formula 1.

This immunoglobulin Fc region may include a hinge region in a heavy chain constant region, but is not limited thereto.

In the present invention, the immunoglobulin Fc region may include a specific hinge sequence at the N-terminus.

As used herein, the term "hinge sequence" refers to a site which is located on a heavy chain and forms a dimer of the immunoglobulin Fc region through an inter-disulfide bond.

In the present invention, the hinge sequence may be mutated to have only one cysteine residue by deletion of a part in the hinge sequence having the following amino acid sequence, but is not limited thereto:
Glu-Ser-Lys-Tyr-Gly-Pro-Pro-Cys-Pro-Ser-Cys-Pro (SEQ ID NO: 103).

The hinge sequence may include only one cysteine residue by deletion of the 8^{th} or 11^{th} cysteine residues in the hinge sequence of SEQ ID NO: 119. The hinge sequence of the present invention may consist of 3 to 12 amino acids including only one cysteine residue, but is not limited thereto. More specifically, the hinge sequence of the present invention may have the following sequences: Glu-Ser-Lys-Tyr-Gly-Pro-Pro-Pro-Ser-Cys-Pro (SEQ ID NO: 104), Glu-Ser-Lys-Tyr-Gly-Pro-Pro-Cys-Pro-Ser-Pro (SEQ ID NO: 105), Glu-Ser-Lys-Tyr-Gly-Pro-Pro-Cys-Pro-Ser (SEQ ID NO: 106), Glu-Ser-Lys-Tyr-Gly-Pro-Pro-Cys-Pro-Pro (SEQ ID NO: 107), Lys-Tyr-Gly-Pro-Pro-Cys-Pro-Ser (SEQ ID NO: 108), Glu-Ser-Lys-Tyr-Gly-Pro-Pro-Cys (SEQ ID NO: 109), Glu-Lys-Tyr-Gly-Pro-Pro-Cys (SEQ ID NO: 110), Glu-Ser-Pro-Ser-Cys-Pro (SEQ ID NO: 111), Glu-Pro-Ser-Cys-Pro (SEQ ID NO: 112), Pro-Ser-Cys-Pro (SEQ ID NO: 113), Glu-Ser-Lys-Tyr-Gly-Pro-Pro-Ser-Cys-Pro (SEQ ID NO: 114), Lys-Tyr-Gly-Pro-Pro-Pro-Ser-Cys-Pro (SEQ ID NO: 115), Glu-Ser-Lys-Tyr-Gly-Pro-Ser-Cys-Pro (SEQ ID NO: 116), Glu-Ser-Lys-Tyr-Gly-Pro-Pro-Cys (SEQ ID NO: 117), Lys-Tyr-Gly-Pro-Pro-Cys-Pro (SEQ ID NO: 118), Glu-Ser-Lys-Pro-Ser-Cys-Pro (SEQ ID NO: 1119), Glu-Ser-Pro-Ser-Cys-Pro (SEQ ID NO: 120), Glu-Pro-Ser-Cys (SEQ ID NO: 121), Ser-Cys-Pro (SEQ ID NO: 122).

More specifically, the hinge sequence may include an amino acid sequence of SEQ ID NO: 113 (Pro-Ser-Cys-Pro) or SEQ ID NO: 122 (Ser-Cys-Pro), but is not limited thereto.

The immunoglobulin Fc region of the present invention may be in the form in which two molecules of the immunoglobulin Fc chain form a dimer due to the presence of a hinge sequence, and the conjugate of Formula 1 of the present invention may be in the form in which one end of the linker is linked to one chain of the dimeric immunoglobulin Fc region, but is not limited thereto.

As used herein, the term "N-terminus" refers to an amino terminus of a protein or polypeptide, and may include the outermost end of the amino terminus, or 1, 2, 3, 4, 5, 6, 7, 8, 9, or 10 or more amino acids from the outermost end. In the immunoglobulin Fc region of the present invention, a hinge sequence may be included in the N-terminus thereof, but is not limited thereto.

The immunoglobulin Fc region of the present invention may be an extended Fc region including a part or the entirety of heavy chain constant region 1 (CH1) and/or light chain constant region 1 (CL1), excluding only heavy chain and light chain variable regions of an immunoglobulin, as long as the immunoglobulin Fc region has a substantially equivalent or improved effect, as compared with the native form. Alternatively, the immunoglobulin Fc region of the present invention may be a region having a deletion of a considerably long partial amino acid sequence corresponding to CH2 and/or CH3.

For example, the immunoglobulin Fc region of the present invention may be 1) a CH1 domain, a CH2 domain, a CH3 domain, and a CH4 domain; 2) a CH1 domain and a CH2 domain; 3) a CH1 domain and a CH3 domain; 4) a CH2 domain and a CH3 domain; 5) a combination of one, or two or more domains among a CH1 domain, a CH2 domain, a CH3 domain, and a CH4 domain and an immunoglobulin hinge region (or a part of the hinge region); and 6) a dimer of each domain of a heavy chain constant region and a light chain constant region, but is not limited thereto.

Further, in an embodiment of the long-acting conjugate of the present invention, the immunoglobulin Fc region F is a dimer consisting of two polypeptide chains, wherein the dimeric Fc region F and X may be covalently linked to each other via one identical linker L containing ethylene glycol repeating units. In a specific embodiment, X is covalently linked to only one polypeptide chain of the two polypeptide chains of the dimeric Fc region F via the linker L. In a more specific embodiment, only one X molecule is covalently linked via L to one polypeptide chain, to which X is linked, of the two polypeptide chains of the dimeric Fc region F. In a most specific embodiment, F is a homodimer.

In another embodiment, the immunoglobulin Fc region F is a dimer consisting of two polypeptide chains, and one end of L may be linked to only one polypeptide chain of the two polypeptide chains, but is not limited thereto.

In another embodiment of the long-acting conjugate of the present invention, two molecules of X may also be symmetrically linked to one Fc region in a dimeric form. In this regard, the immunoglobulin Fc and X may be linked to each other via a non-peptide linker, but is not limited to the above-described embodiments.

Further, the immunoglobulin Fc region of the present invention includes not only the native amino acid sequence as well as a sequence derivative thereof. The amino acid sequence derivative refers to an amino acid sequence which is different from the native amino acid sequence by a deletion, an insertion, or a non-conservative or conservative substitution of one or more amino acid residues, or a combination thereof.

For example, the amino acid residues at positions 214 to 238, 297 to 299, 318 to 322 or 327 to 331, which are known to be important for linkage in IgG Fc, may be used as appropriate sites for variation.

In addition, various types of derivatives are possible by removing a region capable of forming a disulfide bond, deleting some amino acids at the N-terminus of native Fc, or adding a methionine residue at the N-terminus of native Fc. In addition, in order to remove effector functions, a complement-binding site, for example, a C1q-binding site, may be removed, and an antibody-dependent cell-mediated cytotoxicity (ADCC) site may be removed. Techniques for preparing such sequence derivatives of the immunoglobulin Fc region are disclosed in WO 97/34631 and WO 96/32478, etc.

Amino acid exchanges in a protein or peptide that do not alter the entire activity of a molecule are known in the art (H.Neurath, R.L.Hill, The Proteins, Academic Press, New York, 1979). The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, Asp/Gly. In some cases, amino acids may be modified by phosphorylation, sulfation, acrylation, glycosylation, methylation, farnesylation, acetylation, amidation, or the like.

The above-described Fc derivatives exhibit a biological activity equivalent to that of the Fc region of the present invention, and may be obtained by improving the structural stability of the Fc region against heat, pH, etc.

Further, such an Fc region may be obtained from a native form isolated from living bodies of humans or animals, such as cows, goats, pigs, mice, rabbits, hamsters, rats, guinea pigs, etc., or may be a recombinant form obtained from transformed animal cells or microorganisms, or derivatives thereof. Here, a method of obtaining from a native form is a method in which the whole immunoglobulin is isolated from a living body of a human or animal and then treated with a protease. The native form may be digested into Fab and Fc when treated with papain and digested into pF'c and F(ab)2 when treated with pepsin. These may be separated into Fc or pF'c by size-exclusion chromatography or the like. In a more specific embodiment, the human-derived Fc region is a recombinant immunoglobulin Fc region obtained from a microorganism.

In addition, the immunoglobulin Fc region may have native glycans, increased or decreased glycans, as compared with the native form, or be in a deglycosylated form. The increase, decrease, or removal of the immunoglobulin Fc glycans may be attained by using common methods, such as a chemical method, an enzymatic method, and a genetic engineering method using a microorganism. Here, the immunoglobulin Fc region obtained by removal of glycans from Fc shows a significant deterioration in binding affinity to the complement C1q and a decrease or elimination in antibody-dependent cytotoxicity or complement-dependent cytotoxicity, and thus cause no unnecessary immune response *in vivo.* In this regard, a deglycosylated or aglycosylated immunoglobulin Fc region may be a more suitable form to meet the original purpose of the present invention as a drug carrier.

As used herein, the term "deglycosylation" refers to enzymatic elimination of sugars from an Fc region, and the term "aglycosylation" refers to a Fc region that is not glycosylated and produced in prokaryotes, more specifically, in *E. coli.*

Meanwhile, the immunoglobulin Fc region may be originated from humans, or other animals including cows, goats, pigs, mice, rabbits, hamsters, rats, guinea pigs, etc., and in a more specific embodiment, the immunoglobulin Fc region is originated from humans.

In addition, the immunoglobulin Fc region may be an Fc region derived from IgG, IgA, IgD, IgE, IgM, or a combination or hybrid thereof. In a still more specific embodiment, the immunoglobulin Fc region is derived from IgG or IgM, which is most abundant in the human blood, and in a still more specific embodiment, the immunoglobulin Fc region is derived from IgG, which is known to increase the half-life of ligand-binding protein. In a still more specific embodiment, the immunoglobulin Fc region is an IgG4 Fc region, and in a most specific embodiment, the immunoglobulin Fc region is an aglycosylated Fc region derived from human IgG4, but is not limited thereto.

Further, in a specific embodiment, the immunoglobulin Fc fragment is a region of human IgG4 Fc, may be in the form of a homodimer in which two monomers are linked through a disulfide bond (inter-chain form) between cysteines, which are the third amino acid of each monomer. In this regard, each monomer of the homodimer independently have/may have an intra-disulfide bond between cysteines at positions 35 and 95 and an intra-disulfide bond between cysteines at positions 141 and 199, that is, two intra-disulfide bonds (intra-chain form). With respect to the number of amino acids, each monomer may consist of 221 amino acids, and the number of the amino acids forming the homodimer may be a total of 442, but is not limited thereto. Specifically, in the immunoglobulin Fc fragment, two monomers having the amino acid sequence of SEQ ID NO: 123 (consisting of 221 amino acids) form a homodimer through a disulfide bond between cysteines, which are the amino acid at position 3 of each monomer, wherein the monomers of the homodimer independently form an intra-disulfide bond between the cysteines at positions 35 and 95 and an intra-disulfide bond between the cysteines at positions 141 and 199, respectively, but is not limited thereto.

F in Formula 1 may include a monomer having the amino acid sequence of SEQ ID NO: 123, wherein F may be a homodimer of monomers having the amino acid sequence of SEQ ID NO: 123, but is not limited thereto.

In an embodiment, the immunoglobulin Fc region may be a homodimer including the amino acid sequence of SEQ ID NO: 123 (consisting of 442 amino acids), but is not limited thereto.

Meanwhile, as used herein, the "combination", with regard to the immunoglobulin Fc region, means that when a dimer or multimer is formed, a polypeptide encoding the single-chain immunoglobulin Fc region of the same origin forms a bond with a single-chain polypeptide of a different origin. In other words, a dimer or multimer may be prepared from two or more regions selected from the group consisting of IgG Fc, IgA Fc, IgM Fc, IgD Fc, and IgE Fc regions.

As used herein, the "hybrid" means that a sequence corresponding to two or more immunoglobulin Fc regions of different origins is present in a single-chain immunoglobulin constant region. In the present invention, several types of hybrid are possible. In other words, hybridization of domains composed of 1 to 4 domains selected from the group consisting of CH1, CH2, CH3, and CH4 of IgG Fc, IgM Fc, IgA Fc, IgE Fc and IgD Fc is possible, and may include a hinge region.

Meanwhile, IgG may also be divided into IgG1, IgG2, IgG3, and IgG4 subclasses, and a combination or hybridization thereof may also be made in the present invention. Specifically, IgG may be IgG2 and IgG4 subclasses, and more specifically, an Fc fragment of IgG4 having little effector function such as complement dependent cytotoxicity (CDC).

Further, the above-described conjugate may have an increased duration of efficacy, as compared with native GLP1, GIP, or glucagon, or X which is not modified with F, and such a conjugate is not only in the above-described form, but also in the form encapsulated in biodegradable nanoparticles, but is not limited thereto.

Meanwhile, in Formula 1, L is a non-peptide linker, for example, may be a linker containing ethylene glycol repeating units.

In the present invention, the "non-peptide linker" includes a biocompatible polymer having two or more repeating units linked to each other. The repeating units are linked to each other by any covalent linkage but not a peptide linkage. The non-peptide linker may be one element constituting a moiety of the conjugate of the present invention, and corresponds to L in Formula 1.

As the non-peptide linker usable in the present invention, any polymer that has resistance to *in vivo* protease may be used without limitation. In the present invention, the non-peptide linker may be used interchangeably with a non-peptide polymer.

In the present invention, the non-peptide linker includes reactive groups at the ends thereof and thus may form a conjugate by reaction with other elements constituting the conjugate. When a non-peptide linker having reactive functional groups at both ends thereof binds to X and F in Formula 1 through the respective reactive groups to form a conjugate, the non-peptide linker or non-peptide polymer may be named a non-peptide polymer linker moiety or a non-peptide linker moiety.

Although not particularly limited to, the non-peptide linker may be a linker containing ethylene glycol repeating units, for example, polyethylene glycol, and derivatives thereof already known in the art and derivatives that may be easily prepared within the level of skill in the art also fall within the scope of the present invention.

The repeating units of the non-peptide linker may be ethylene glycol repeating units, and specifically, the non-peptide linker may include, at ends thereof, functional groups for use in the preparation of the conjugate, while including ethylene glycol repeating units. The long-acting conjugate according to the present invention may be in the form in which X and F are linked through the functional groups, but is not limited thereto. In the present invention, the non-peptide linker may include two, or three or more functional groups, and the respective functional groups may be the same as or different from each other, but are not limited thereto.

In a specific embodiment, L which is the linker containing the ethylene glycol repeating units may include, at ends thereof, functional groups for use in the preparation of the conjugate before being configured into the conjugate. The long-acting conjugate according to the present invention may be in the form in which X and F are linked through the functional groups, but is not limited thereto. In the present invention, the linker containing the ethylene glycol repeating units may include two, or three or more functional groups, and respective functional groups may be the same as or different from each other, but are not limited thereto.

Specifically, the linker may be polyethylene glycol (PEG) represented by Formula 2 below, but is not limited thereto: 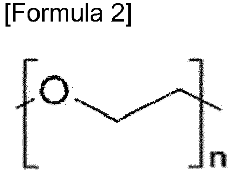 wherein n is 10 to 2400, n is 10 to 480, or n is 50 to 250, but is not limited thereto.

In the long-acting conjugate, the PEG moiety may include not only the - (CH₂CH₂O)ₙ- structure, but also an oxygen atom interposed between a linking element and the -(CH₂CH₂O)ₙ-, but is not limited thereto.

Further, in a specific embodiment, the conjugate may have a structure, in which the peptide (X) including any one amino acid sequence of SEQ ID NOS: 1 to 102, and the immunoglobulin Fc region (F) may be covalently linked via the linker containing ethylene glycol repeating units, but is not limited thereto.

The polyethylene glycol is a term encompassing all of the forms of ethylene glycol homopolymers, PEG copolymers, and monomethyl-substituted PEG polymers (mPEG), but is not particularly limited thereto.

Further, in a specific embodiment, the ethylene glycol repeating unit may be represented by, for example, [OCH₂CH₂]ₙ, wherein the value of n is a natural number, and an average molecular weight, for example, a number average molecular weight of the moiety of [OCH₂CH₂]ₙ in the peptide conjugate may be set to more than 0 kDa to about 100 kDa, but is not limited thereto. As another example, the value of n is a natural number, and the average molecular weight, for example, the number average molecular weight of the moiety of [OCH₂CH₂]ₙ in the peptide conjugate may be about 1 kDa to about 100 kDa, about 1 kDa to about 80 kDa, about 1 kDa to about 50 kDa, about 1 kDa to about 30 kDa, about 1 kDa to about 25 kDa, about 1 kDa to about 20 kDa, about 1 kDa to about 15 kDa, about 1 kDa to about 13 kDa, about 1 kDa to about 11 kDa, about 1 kDa to about 10 kDa, about 1 kDa to about 8 kDa, about 1 kDa to about 5 kDa, about 1 kDa to about 3.4 kDa, about 3 kDa to about 30 kDa, about 3 kDa to about 27 kDa, about 3 kDa to about 25 kDa, about 3 kDa to about 22 kDa, about 3 kDa to about 20 kDa, about 3 kDa to about 18 kDa, about 3 kDa to about 16 kDa, about 3 kDa to about 15 kDa, about 3 kDa to about 13 kDa, about 3 kDa to about 11 kDa, about 3 kDa to about 10 kDa, about 3 kDa to about 8 kDa, about 3 kDa to about 5 kDa, about 3 kDa to about 3.4 kDa, about 8 kDa to about 30 kDa, about 8 kDa to about 27 kDa, about 8 kDa to about 25 kDa, about 8 kDa to about 22 kDa, about 8 kDa to about 20 kDa, about 8 kDa to about 18 kDa, about 8 kDa to about 16 kDa, about 8 kDa to about 15 kDa, about 8 kDa to about 13 kDa, about 8 kDa to about 11 kDa, about 8 kDa to about 10 kDa, about 9 kDa to about 15 kDa, about 9 kDa to about 14 kDa, about 9 kDa to about 13 kDa, about 9 kDa to about 12 kDa, about 9 kDa to about 11 kDa, about 9.5 kDa to about 10.5 kDa, or about 10 kDa, but is not limited thereto.

In a specific embodiment, both ends of the linker may be linked to a thiol group, an amino group, or a hydroxyl group of the immunoglobulin Fc region and a thiol group, an amino group, an azide group, or a hydroxyl group of the peptide, respectively, but are not limited thereto.

Specifically, the linker may include, at both ends thereof, reactive groups capable of binding to the immunoglobulin Fc region and the peptide, respectively, specifically, reactive groups capable of binding to a thiol group of cysteine; an amino group located at the N-terminus, lysine, arginine, glutamine, and/or histidine; and/or a hydroxyl group located at the C-terminus in the immunoglobulin Fc region; and a thiol group of cysteine; an amino group of lysine, arginine, glutamine, and/or histidine; an azide group of azido-lysine; and/or a hydroxyl group in the peptide, but is not limited thereto.

More specifically, each of the reactive groups of the linker may be one or more selected from the group consisting of an aldehyde group, a maleimide group, and a succinimide derivative, but is not limited thereto.

In the above, the aldehyde group may be exemplified by a propionaldehyde group or a butyraldehyde group, but is not limited thereto.

In a specific embodiment, both ends of the non-peptide linker may bind to an amine group or a thiol group of F, e.g., immunoglobulin Fc region and an amine group or a thiol group of X, respectively.

Specifically, the non-peptide polymer may include, at both ends thereof, reactive groups capable of binding to F (e.g., immunoglobulin Fc region) and X, respectively, specifically, reactive groups capable of binding to an amine group located at the N-terminus or lysine, or a thiol group of cysteine of X or F (e.g., immunoglobulin Fc region), but is not limited thereto.

Further, the reactive groups of the non-peptide polymer, capable of binding to F, e.g., immunoglobulin Fc region, and X, may be selected from the group consisting of an aldehyde group, a maleimide group, and a succinimide derivative, but are not limited thereto.

In the above, the aldehyde group may be exemplified by a propionic aldehyde group or a butyl aldehyde group, but is not limited thereto.

In the above, the succinimide derivative may be exemplified by succinimidyl valerate, succinimidyl methylbutanoate, succinimidyl methylpropionate, succinimidyl butanoate, succinimidyl propionate, N-hydroxysuccinimide, hydroxy succinimidyl, succinimidyl carboxymethyl, and succinimidyl carbonate, but is not limited thereto.

The non-peptide linker may be linked to X and F via such reactive groups to be converted into a non-peptide linker moiety, but is not particularly limited thereto.

In addition, a final product produced by reductive amination through aldehyde linkage is still more stable than those obtained through amide linkage. The aldehyde reactive group selectively reacts with the N-terminus at low pH while forming a covalent linkage with a lysine residue at high pH, for example, at pH 9.0.

In addition, the reactive groups of both ends of the non-peptide linker may be the same as or different from each other, and for example, aldehyde groups may be provided at both ends, or a maleimide group may be provided at one end and an aldehyde group, a propionaldehyde group, or a butyraldehyde group may be provided at the other end. However, the reactive groups are not particularly limited thereto as long as F, specifically, the immunoglobulin Fc region, and X may be linked to both ends of the non-peptide linker, respectively.

For example, the non-peptide linker may include a maleimide group as a reactive group at one end and an aldehyde group, a propionaldehyde group, a butyraldehyde group, or the like at the other end.

When polyethylene glycol having hydroxyl reactive groups at both ends is used as a non-peptide polymer, the long-acting protein conjugate of the present invention may be prepared by activating the hydroxyl groups into various reactive groups through known chemical reactions or by using commercially available polyethylene glycol having modified reactive groups.

In a specific embodiment, the non-peptide polymer may be linked to a cysteine residue of X, more specifically, a -SH group of cysteine, but is not limited thereto.

For example, the non-peptide polymer may be linked to the cysteine residue at position 10, the cysteine residue at position 13, the cysteine residue at position 15, the cysteine residue at position 17, the cysteine residue at position 19, the cysteine residue at position 21, the cysteine residue at position 24, the cysteine residue at position 28, the cysteine residue at position 29, the cysteine residue at position 30, the cysteine residue at position 31, the cysteine residue at position 40, or the cysteine residue at position 41 in the peptide corresponding to X, but is not particularly limited.

Specifically, a reactive group of the non-peptide polymer may be linked to the -SH group of the cysteine residue, and all of those described above are applied to the reactive group. When maleimide-PEG-aldehyde is used, the maleimide group may be linked to the -SH group of X through a thioether linkage, and the aldehyde group may be linked to the -NH₂ group of F, specifically, the immunoglobulin Fc region through reductive amination, but is not limited thereto, and this corresponds to one example.

Through such reductive alkylation, an amino group at the N-terminus of the immunoglobulin Fc region is linked to an oxygen atom located at one end of PEG which is the non-peptide polymer via a linker reactive group having a structure of - CH₂CH₂CH₂- to form a structure, like -PEG-O-CH2CH2CH2NH-immunoglobulin Fc, and through a thioether linkage, a structure in which one end of PEG is linked to a sulfur atom located at cysteine of the peptide including any one sequence of SEQ ID NOS: 1 to 102 may be formed. The above-described thioether linkage may include a structure of

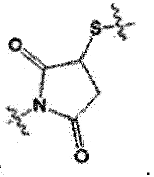

However, the non-peptide polymer is not particularly limited to the above embodiment, and this corresponds to one example.

In another specific embodiment, the non-peptide polymer may be linked to a lysine residue of, more specifically, an amino group of lysine of X, but is not limited thereto.

Further, in the conjugate, the reactive group of the non-peptide polymer may be linked to -NH₂ located at the N-terminus of the immunoglobulin Fc region, but this corresponds to one example.

Further, in the conjugate, the peptide may be linked to a linker having a reactive group through the C-terminus thereof, but this corresponds to one example.

As used herein, the term "C-terminus" refers to a carboxy terminus of the peptide, and with respect to the objects of the present invention, the term refers to a position that may be linked to a linker. For example, although not limited, the C-terminus may include not only the amino acid residue at the outermost end of the C-terminus but also amino acid residues near the C-terminus, specifically, the 1^{st} to 20^{th} amino acid residues from the outermost end, but is not limited thereto.

F is an immunoglobulin Fc region, and includes not only the native sequence obtained by papain digestion of immunoglobulins, but also derivatives thereof, substituents thereof, for example, variants in which one or more amino acid residues in the native sequence are modified by deletion, insertion, non-conservative or conservative substitution, or a combination thereof and are thus different from that of the native form. The derivatives, substituent, and variants are required to retain FcRn-binding ability.

F has a structure in which two polypeptide chains are linked by a disulfide bond, wherein the two polypeptide chains are linked through only a nitrogen atom of one of the two chains, but are not limited thereto. The linking through a nitrogen atom may be linking to the epsilon amino atoms of lysine or the N-terminus amino group through reductive amination, but is not limited thereto.

The reductive amination refers to a reaction in which an amine group or an amino group of one reactant reacts with an aldehyde (i.e., a functional group capable of reductive amination) of another reactant to form an amine, and then an amine linkage is formed by reductive reaction, and reductive amination is an organic synthetic reaction widely known in the art.

In a specific embodiment, F may be linked through a nitrogen atom of the N-terminus proline thereof, but is not limited thereto.

Unless indicated otherwise herein, the contents in the detailed description and claims with respect to the "peptide" according to the present invention or the "conjugate" in which this peptide is covalently linked to a biocompatible material through a covalent linkage are applied to the form of not only the corresponding peptide or conjugate but also a salt of the corresponding peptide or conjugate (e.g., a pharmaceutically acceptable salt of the peptide), or a solvate thereof. Therefore, although described as "peptide" or "conjugate" herein, the corresponding described contents are equally applied to a specific salt thereof, a specific solvate thereof, and a specific solvate of the specific salt thereof. These salts may be in the form in which, for example, any pharmaceutically acceptable salt is used. The type of the salt is not particularly limited. However, the salt is preferably in the form that is safe and effective to a subject, e.g., a mammal, but is not particularly limited thereto.

The type of the salt is not particularly limited. However, the salt is preferably in the form that is safe and effective to a subject, e.g., a mammal, but is not particularly limited thereto.

The term "pharmaceutically acceptable" refers to a substance that may be effectively used for a desired purpose without causing excessive toxicity, irritation, allergic responses, etc. within the range of the medical and pharmaceutical decision.

As used herein, the term "pharmaceutically acceptable salt" refers to a salt derived from pharmaceutically acceptable inorganic acids, organic acids, or bases. Examples of appropriate acids may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, etc. Salts derived from appropriate bases may include alkali metals such as sodium, potassium, etc., alkali earth metals such as magnesium, etc., ammonium, etc.

Further, as used herein, the term "solvate" refers to a complex formed between the peptide according to the present invention or a salt thereof and a solvent molecule.

In the present invention, the SGLT-2 inhibitor which is administered in combination with the peptide or the conjugate thereof is also called sodium-glucose cotransporter 2, and may be any one or more selected from the group consisting of empagliflozin, dapagliflozin, canagliflozin, remogliflozin, remogliflozin etabonate, sergliflozin, ipragliflozin, tofogliflozin, luseogliflozin, sotagliflozin, bexagliflozin, atigliflozin, and ertugliflozin, but is not limited thereto.

It is known that the SGLT-2 is involved in the reabsorption of glucose in the proximal tubules, and excessive glucose reabsorption occurs due to SGLT-2 in diabetic patients. The blood glucose lowering effect may be obtained by administering the peptide according to the present invention or the conjugate thereof in combination with the SGLT-2 inhibitor.

As used herein, the "diabetes" is a kind of metabolic disease in which insulin secretion is insufficient or normal functions are not made. Diabetes is characterized by hyperglycemia in which blood glucose levels are elevated, and is a disease which causes various conditions and syndromes due to hyperglycemia, and excretes glucose with urine. Diabetes may be divided into type 1 diabetes and type 2 diabetes, and as long as not only the blood glucose lowering effect but also the weight loss effect may be achieved, the peptide according to the present invention or the conjugate thereof may be administered in combination with the SGLT-2 inhibitor.

In the present invention, diabetes may include obese diabetes and diabetic complications.

The "obese diabetes" refers to diabetes accompanied by symptoms of obesity that are the cause of diabetes, especially, type 2 diabetes, or symptoms of obesity that are generally accompanied in type 2 diabetic patients. It is known that approximately 80% to 90% of patients with type 2 diabetes have symptoms of obesity. In the present invention, the obese diabetes may be caused by obesity.

The "diabetic complications" refer to various pathological symptoms that occur in the body as hyperglycemia is maintained for a long period of time, and are exemplified by stroke, vascular complications, heart attack, diabetic retinopathy, renal dysfunction, chronic kidney disease, neuropathy, diabetic foot ulceration, and cardiovascular disease, but are not limited thereto. When hyperglycemia is maintained for a long period of time, the risk of diabetes complications increases, and thus effective blood glucose management and weight control are necessary to prevent these complications.

In the present invention, obesity refers to excessive body weight (body mass index of 25.0 or more) and is also a major cause of diabetes. Since obesity is closely related to diabetes, weight loss has significance as a treatment for obesity, and also has important significance in the treatment of diabetes. Since not only the blood glucose lowering effect but also weight loss effect may be obtained by administering the peptide of the present invention or the conjugate thereof in combination with the SGLT-2 inhibitor, the composition including the peptide or the conjugate thereof which is administered in combination with the SGLT-2 inhibitor may be used to prevent or treat obesity, specifically, to treat obese patients with hyperglycemia or diabetes. Alternatively, excellent prophylactic or therapeutic effects may be obtained in diabetic patients, particularly, diabetic patients with overweight or obesity.

Accordingly, the composition including the peptide or the conjugate thereof which is administered in combination with the SGLT-2 inhibitor of the present invention may be administered to patients with diabetes and/or obesity, for example, diabetic patients with overweight or obesity; or obese patients with diabetes to exhibit the blood glucose lowering and weight loss effects, thereby having the effects of preventing or treating diabetes and/or obesity, but is not limited thereto.

Meanwhile, the effect by such combination administration may exert on patients with various diseases caused by weight gain and blood glucose dysregulation, e.g., metabolic syndromes, as well as on patients with diabetes and obesity.

In the present invention, the metabolic syndromes collectively refer to several metabolism-related diseases. Examples of the metabolic syndromes may include, but are not limited to, diabetes, obesity, hyperlipidemia, dyslipidemia, etc. Representative causes of these metabolic syndromes may include insulin resistance, overweight, etc.

Diabetes and obesity are as described above.

In the present invention, hyperlipidemia refers to a disease in which low-density lipoprotein cholesterols and/or triglycerides are elevated in the blood. For example, low-density lipoprotein cholesterol of more than 130 mg/dL and/or triglyceride of more than 200 mg/dL may corresponding to hyperlipidemia.

In the present invention, dyslipidemia refers to a condition in which blood levels of low-density lipoprotein cholesterol, high-density lipoprotein cholesterol, and triglyceride exceed the normal range.

Since these metabolic syndromes are known to be closely related to hyperglycemia and weight gain, the blood glucose lowering and weight loss effects may be achieved through combination administration according to the present invention, thereby obtaining the effects of preventing or treating the target syndrome.

Further, since the composition including the peptide or the conjugate thereof, which is administered in combination with the SGLT-2 inhibitor according to the present invention, also has anti-inflammatory and anti-fibrotic effects, it may exhibit the effects of preventing or treating liver disease, lung disease, or respiratory infections.

In the present invention, the liver disease may include simple steatosis, non-alcoholic fatty liver disease, liver inflammation, non-alcoholic steatohepatitis, metabolic liver disease, cholestatic liver disease, liver fibrosis, cirrhosis, liver failure, liver cancer, etc., but is not limited thereto.

In the present invention, the lung disease may include interstitial lung disease, progressive fibrosing interstitial lung disease, idiopathic interstitial pneumonia, non-specific interstitial pneumonia, pulmonary fibrosis, interstitial pulmonary fibrosis, idiopathic pulmonary fibrosis, alveolitis, pneumonia, emphysema, bronchitis, and chronic obstructive pulmonary disease (COPD), combined pulmonary fibrosis and emphysema (CPFE), asthma, etc.

With regard to the liver and lung diseases, the main pathological mechanisms may include the occurrence of inflammation, tissue damage, and resulting fibrosis. Since the composition including the peptide or the conjugate thereof, which is administered in combination with the SGLT-2 inhibitor according to the present invention, has anti-inflammatory and anti-fibrotic effects, it may also show the effects on liver disease and lung disease.

The respiratory infections of the present invention are respiratory diseases caused by infection with pathogens (viruses, bacteria, fungi, etc.), and a representative cause of infection is a respiratory virus. Among respiratory infections, respiratory viral infection refers to a respiratory disease caused by pathogenic viral infections. The respiratory virus may include adenovirus, vaccinia virus, herpes simplex virus, parainfluenza virus, rhinovirus, varicella zoster virus, measle virus, respiratory syncytial virus, Dengue virus, human immunodeficiency virus (HIV), influenza virus, coronavirus, severe acute respiratory syndrome associated virus (SARS-associated virus), or middle east respiratory syndrome coronavirus (MERS-CoV), but is not limited thereto.

Non-limiting example of the coronavirus may include SARS-CoV-2, and infection with SARS-CoV-2 may cause coronavirus disease-19 (coronavirus disease 2019, COVID-19).

As used herein, the term "coronavirus disease-19" is a viral infectious disease caused by coronavirus (2019-nCoV or SARS-CoV-2) infection. Although the clear source and route of infection have not yet been identified, it is highly contagious and has caused a global pandemic.

The respiratory infections of the present invention may include sequelae of respiratory infections.

The sequelae of respiratory infections of the present invention refers to abnormal symptoms that appear independently of respiratory infections in patients with respiratory infections. Specifically, in the present invention, the sequelae of respiratory infections may refer to sequelae of respiratory viral infection, and more specifically, sequelae of coronavirus infection-19 (COVID-19), but is not limited thereto.

As used herein, the term "coronavirus disease-19 (COVID-19) sequelae" refers to sequelae that appear in patients after SARS-CoV-2 infection. Specifically, the coronavirus disease-19 sequelae may include shortness of breath, cough, pneumonia, pulmonary fibrosis, pain, inflammation, nervous system disorders, etc., but are not limited thereto. Shortness of breath, cough, chronic fatigue, pain, pneumonia, fibrosis, etc. that appear as sequelae of COVID-19 may be caused by lowered function and/or damage of the respiratory system, particularly, of the lungs, but are not limited to this.

As used herein, the term "preventing" refers to all actions that inhibit or delay metabolic syndrome, liver disease, lung disease, or respiratory infections by administering the peptide (e.g., the peptide itself or the long-acting conjugate in which a biocompatible material is conjugated to the peptide) or the composition including the same in combination with the SGLT-2 inhibitor. As used herein, the term "treating" refers to all actions that improve or advantageously change the symptoms of metabolic syndrome, liver disease, lung disease, or respiratory infections by administering the peptide (e.g., the peptide itself or the long-acting conjugate in which a biocompatible material is conjugated to the peptide) or the composition including the same in combination with the SGLT-2 inhibitor.

The composition according to the present invention may include the peptide (triple agonist) or the conjugate thereof, and specifically, the peptide or the conjugate thereof in a pharmaceutically effective amount. Further, the composition may further include a pharmaceutically acceptable excipient. The composition of the present invention may have use in preventing or treating metabolic syndrome, liver disease, lung disease, or respiratory infections, or may have use in lowering blood glucose, but is not limited thereto. Additionally, the composition of the present invention may have use in reducing body weight.

As used herein, the term "pharmaceutical acceptable" refers to a sufficient amount to show therapeutic effects without causing side effects, and the amount may be easily determined by a person skilled in the art according to factors that are well known in the medical field, including the type of disease, a patient's age, body weight, health condition, and sex, the sensitivity of a patient to drugs, the route of administration, the method of administration, the number of times of administration, the period of treatment, drugs used in combination or at the same time, and the like.

Regarding the pharmaceutically acceptable excipient, a binder, a lubricant, a disintegrant, an excipient, a solubilizer, a dispersant, a stabilizer, a suspending agent, a coloring agent, a flavoring agent, etc. may be used for oral administration; a buffer, a preservative, an analgesic, a solubilizer, an isotonic agent, a stabilizer, etc. may be used in combination for injectable preparations; and a base, an excipient, a lubricant, a preservative, etc. may be used for topical administration. The formulations of the composition of the present invention may be prepared in various manners by mixing with the pharmaceutically acceptable excipient described above. For example, for oral administration, the composition may be formulated in the form of a tablet, a troche, a capsule, an elixir, a suspension, a syrup, a wafer, etc.; and for injections, the pharmaceutical composition may be formulated in the form of a unit-dosing ampoule or a multi-dosing form. In addition, the composition may also be formulated into a solution, a suspension, a tablet, pills, a capsule, a sustained-release preparation, etc.

Meanwhile, examples of a carrier, an excipient, and a diluent suitable for the formulation may include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oils, etc. In addition, a filler, an anti-coagulant, a lubricant, a wetting agent, a flavor, an emulsifier, a preservative, etc. may be further included.

Further, the composition of the present invention may have any one formulation selected from the group consisting of a tablet, a pill, a powder, granules, a capsule, a suspension, a liquid preparation for internal use, an emulsion, a syrup, a sterile aqueous solution, a non-aqueous solvent, a lyophilized preparation, and a suppository.

Further, the peptide or conjugate may be used by mixing with various carriers approved as drugs, such as physiological saline or organic solvents, and to increase stability or absorptivity, carbohydrates such as glucose, sucrose, or dextran, antioxidants such as ascorbic acid or glutathione, chelating agents, low-molecular-weight proteins, or other stabilizers may be used as medical agents.

The dose and frequency of the composition of the present invention are determined according to the type of drug as an active ingredient, along with various factors, such as a disease to be treated, a route of administration, a patient's age, sex, and body weight, and severity of a disease. Specifically, the composition of the present invention may include the peptide or the conjugate thereof in a pharmaceutically effective amount, but is not limited thereto.

Including the peptide or the conjugate thereof in a pharmaceutically effective amount refers to a level at which desired pharmaceutical activity (e.g., lowered blood glucose and/or weight loss) may be obtained by the peptide or the conjugate thereof, and may also refer to a level at which toxicities or side effects do not occur or occur at an insignificant level or a pharmaceutically acceptable level in a subject to be administered, but is not limited thereto. Such a pharmaceutically effective amount may be determined by comprehensively considering the frequency of administration, patient, formulations, etc.

Although not particularly limited, the pharmaceutical composition of the present invention may include the ingredient (active ingredient) in an amount of 0.01% (w/v) to 99% (w/v).

The total effective dose of the composition of the present invention may be administered to a patient in a single dose, or may be administered in multiple doses according to a fractionated treatment protocol for a long period of time. In the composition of the present invention, the content of an active ingredient may vary according to the severity of a disease. Specifically, the preferable total daily dose of the peptide of the present invention or the long-acting conjugate thereof may be about 0.0001 mg to 500 mg per 1 kg of body weight of a patient. However, the effective dose of the peptide or the long-acting conjugate thereof is determined considering various factors including a patient's age, body weight, health condition, sex, severity of a disease, a diet, and an excretion rate, in addition to the route of administration and the frequency of treatment of the pharmaceutical composition. Therefore, considering this, a person skilled in the art may easily determine an appropriate effective dose according to particular uses of the composition of the present invention. The pharmaceutical composition according to the present invention is not particularly limited to the formation, route of administration, and method of administration, as long as the pharmaceutical composition shows the effects according to the present invention.

For example, the composition of the present invention may be administered once every 3 days, once a week, once every 2 weeks, once every 4 weeks, or once a month, but is not limited thereto.

The composition of the present invention may be administered to mammals including rats, livestock, etc., including humans in need of lowering blood glucose and/or weight loss, specifically, patients with diabetes and/or obesity, and more specifically, diabetic patients with overweight or obesity, or obese patients with hyperglycemia or diabetes, but there are no limitations on the subjects of administration as long as beneficial effects may be obtained by lowering blood glucose and reducing weight. Further, the composition of the present invention may be administered to patients with metabolic syndrome, liver disease, lung disease, or respiratory infections, but is not limited thereto.

The peptide of the present invention or the long-acting conjugate thereof; or the composition including the same may be administered in combination with the SGLT-2 inhibitor, and due to the combination administration, weight loss and blood glucose lowering effects may be obtained, thereby exhibiting the excellent prophylactic or therapeutic effects on diabetic and/or obese patients, particularly, diabetic patient with overweight or obesity, or obese patients with hyperglycemia or diabetes.

Alternatively, the peptide of the present invention or the long-acting conjugate thereof which may be administered in combination with the SGLT-2 inhibitor; or the composition including the same has anti-inflammatory and/or anti-fibrotic effects, thereby exhibiting the excellent prophylactic or therapeutic effects on patients with liver disease, lung disease, or respiratory infections.

The SGLT-2 inhibitor may have a formulation suitable for combination administration, for example, any one formulation selected from the group consisting of a tablet, a pill, a powder, granules, a capsule, a suspension, a liquid preparation for internal use, an emulsion, a syrup, a sterile aqueous solution, a non-aqueous solvent, a lyophilized preparation, and a suppository, but is not limited thereto. Further, the SGLT-2 inhibitor may be administered via an administration route suitable for combination administration, for example, via an oral or parenteral route, such as through skin, intravenously, intramuscularly, intraarterially, intramedullarily, intrathecally, intraventricularly, pulmonarily, transdermally, subcutaneously, intraperitoneally, intranasally, intragastrically, topically, sublingually, vaginally, or rectally, but is not limited thereto.

The peptide of the present invention or the long-acting conjugate thereof may be administered in combination with the SGLT-2 inhibitor at an appropriate ratio. For example, based on one dose, the peptide of the present invention or the long-acting conjugate thereof may be administered in an amount of 0.1 mg to 100 mg, 0.5 mg to 80 mg, 1 mg to 60 mg, 1 mg to 25 mg, 5 mg to 20 mg, or 10 mg to 15 mg, and the SGLT-2 inhibitor may be administered in an amount of 1 mg to 50 mg, 3 mg to 30 mg, or 5 mg to 25 mg, but are not limited thereto.

When the peptide of the present invention or the long-acting conjugate thereof may be administered in combination with the SGLT-2 inhibitor at an appropriate ratio, it may exhibit the weight loss effect while exhibiting the excellent blood glucose control effect. When the peptide or the long-acting conjugate thereof is excessively administered, there is a high risk of not having the blood glucose control effect at a level that can treat diabetic patients, and when the SGLT-2 inhibitor is excessively administered, there is a high risk of side effects such as weight gain.

The peptide of the present invention or the long-acting conjugate thereof; or the composition including the same may be administered, together with the SGLT-2 inhibitor, at regular intervals, or may be administered individually at different frequencies, and the time of administration is not limited as long as the effects of combination administration of the two components are achieved.

Although not limited to, the frequency of administration of the peptide of the present invention or the long-acting conjugate thereof, or the composition including the same; and the SGLT-2 inhibitor may be independently administered once every 3 days, once a week, once every 2 weeks, once every 4 weeks, or once a month, and the administration interval between the peptide of the present invention or the long-acting conjugate thereof, or the composition including the same; and the SGLT-2 inhibitor may be 1 day to 10 days, 1 day to 5 days, or 1 day to 3 days, but is not limited thereto.

The above description may also be applied to other embodiments or other aspects of the present invention, but is not limited thereto.

In accordance with another aspect of the present invention, there is provided a combination, pharmaceutical composition, or kit including the peptide having activities to glucagon receptor, GLP-1 receptor, and GIP receptor, or the conjugate thereof; and the SGLT-2 inhibitor. In an embodiment, there is provided a combination, pharmaceutical composition, or kit for preventing or treating liver disease, lung disease, or respiratory infections. In another embodiment, there is provided a combination, pharmaceutical composition, or kit for preventing or treating diabetes and/or obesity.

The peptide, conjugate thereof, composition, liver disease, lung disease, or respiratory infections, diabetes, obesity, SGLT-2 inhibitor, combination administration, preventing, and treating are as described above.

The composition, combination, or kit of the present invention may include (i) the peptide having activities to glucagon receptor, GLP-1 receptor, and GIP receptor, or (ii) the long-acting conjugate of the peptide having activities to glucagon receptor, GLP-1 receptor, and GIP receptor, and additionally/optionally, the SGLT-2 inhibitor, but is not limited thereto. The composition, combination, and kit of the present invention may be administered to/used in patients with metabolic syndrome, liver disease, lung disease, or respiratory infections, thereby exhibiting prophylactic or therapeutic effects, but are not limited thereto. Alternatively, the composition, combination, and kit of the present invention may be administered to/used in diabetic patient, obese patients, diabetic patients with overweight or obesity, or obese patients with diabetes, thereby exhibiting the blood glucose lowering and weight loss effects, but are not limited thereto.

As used herein, the term "combination" has use of combination administration of the peptide having activities to glucagon receptor, GLP-1 receptor, and GIP receptor or the conjugate thereof; and the SGLT-2 inhibitor, and may be understood as having the same meaning as "combined used". It may include a form of a pharmaceutical composition, which is characterized in that the peptide having activities to glucagon receptor, GLP-1 receptor, and GIP receptor; and the SGLT-2 inhibitor are used in combination, but is not limited thereto.

The combination may be administered
a) in a single mixture, in which (i) the peptide having activities to glucagon receptor, GLP-1 receptor, and GIP receptor, or the conjugate thereof; and (ii) the SGLT-2 inhibitor are mixed; or
b) in a separate form, in which (i) the peptide having activities to glucagon receptor, GLP-1 receptor, and GIP receptor, or the conjugate thereof; and (ii) the SGLT-2 inhibitor are separated, but is not limited thereto.

When the peptide having activities to glucagon receptor, GLP-1 receptor, and GIP receptor, or the conjugate thereof, and the SGLT-2 inhibitor are in a separate form, the substance having activities to glucagon receptor, GLP-1 receptor, and GIP receptor, or the conjugate thereof, and the SGLT-2 inhibitor are formulated into separate preparations, which may be administered simultaneously, separately, sequentially, or reverse sequentially.

The composition of the present invention including the peptide having activities to glucagon receptor, GLP-1 receptor, and GIP receptor, or the conjugate thereof, which is administered in combination with the SGLT-2 inhibitor, may include the combination.

As used herein, the term "composition including the combination" may be the combination itself including the peptide having activities to glucagon receptor, GLP-1 receptor, and GIP receptor, or the conjugate thereof, and the SGLT-2 inhibitor, or the composition including the same and having the therapeutic use, but is not limited thereto. For example, the composition may have use in preventing or treating metabolic syndrome, liver disease, lung disease, or respiratory infections, but is not limited thereto. Specifically, the composition may have use in preventing or treating diabetes and/or obesity, but is not limited thereto. As used herein, the "composition including the combination" may be used interchangeably with the "composition".

The composition including the combination according to the present invention is for combination administration of the peptide having activities to glucagon receptor, GLP-1 receptor, and GIP receptor, or the conjugate thereof, and the SGLT-2 inhibitor, in which the peptide having activities to glucagon receptor, GLP-1 receptor, and GIP receptor, or the conjugate thereof; and the SGLT-2 inhibitor are formulated into a single preparation or individually formulated. Specifically, the peptide having activities to glucagon receptor, GLP-1 receptor, and GIP receptor, or the conjugate thereof; and the SGLT-2 inhibitor may be administered simultaneously, separately, sequentially, or reverse sequentially, but are not limited thereto.

As used herein, the term "kit" may include the peptide having activities to glucagon receptor, GLP-1 receptor, and GIP receptor according to the present invention, or the conjugate thereof; and the SGLT-2 inhibitor for combination administration of the peptide having activities to glucagon receptor, GLP-1 receptor, and GIP receptor, or the conjugate thereof; and the SGLT-2 inhibitor. Specifically, the kit according to the present invention may include the peptide having activities to glucagon receptor, GLP-1 receptor, and GIP receptor, or the conjugate thereof; and the SGLT-2 inhibitor which are formulated into a single preparation, or may include individual preparations of the peptide, or the conjugate thereof; and the SGLT-2 inhibitor, and may further include substances needed for the combination administrations of the two substances, but is not limited thereto.

Due to administration/use of the combination, composition, or kit of the present invention, it is possible to obtain the weight loss and blood glucose lowering effects, thereby obtaining the excellent effect of preventing or treating diabetes and/or obesity in patients with metabolic syndrome, specifically, diabetic patients, particularly, diabetic patients with overweight or obesity; or obese patients, particularly, obese patients with diabetes.

Further, due to administration/use of the combination, composition, or kit of the present invention, it is possible to obtain anti-inflammatory and/or anti-fibrotic effects as well as the weight loss and blood glucose lowering effects, thereby obtaining the prophylactic or therapeutic effects in patients with liver disease, lung disease, or respiratory infections.

The combination, composition, or kit of the present invention may be used for combination administration of the peptide having activities to glucagon receptor, GLP-1 receptor, and GIP receptor, or the conjugate thereof; and the SGLT-2 inhibitor.

In accordance with still another aspect of the present invention, there is provided a method of preventing or treating diabetes and/or obesity or a method of lowering blood glucose, the method including the step of administering the peptide, the conjugate thereof, or the composition including the same to a subject in need thereof.

In accordance with still another aspect of the present invention, there is provided a method of preventing or treating metabolic syndrome, liver disease, lung disease, or respiratory infections or a method of lowering blood glucose, the method including the step of administering the peptide, the conjugate thereof, or the composition including the same to a subject in need thereof.

The method of the present invention may further include the step of administering the SGLT-2 inhibitor to a subject in need thereof. For specific example, the method of the present invention may be a method of administering the peptide or the conjugate thereof in combination with the SGLT-2 inhibitor, but is not limited thereto.

The peptide, conjugate thereof, composition, SGLT-2 inhibitor, combination administration, diabetes, obesity, metabolic syndrome, liver disease, lung disease, or respiratory infections, preventing, treating, and blood glucose lowering are as described above.

As used herein, the subject refers to a subject for which blood glucose lowering and weight loss are beneficial, and means a mammal including a rat, livestock, and the like, including a human being, but any subject for which the blood glucose lowering effect by the peptide of the present invention or the conjugate thereof, or the composition including the same is beneficial is included without limitation. In particular, the subject may be a subject having diabetes and/or obesity, but is not limited thereto. In a specific embodiment, the subject may be a diabetic patient with overweight or obesity, or a subject having or at risk of having obese diabetes or diabetic complications, but is not limited thereto. Alternatively, the subject may be an obese subject or an obese subject with hyperglycemia or diabetes, but is not limited thereto. Alternatively, the subject may be a subject with metabolic syndrome, liver disease, lung disease, or respiratory infections, but is not limited thereto.

As used herein, the term "administration" refers to the introduction of a predetermined substance to a patient (subject) by any suitable method, and the route of administration of the peptide, the conjugate thereof, or composition; and the SGLT-2 inhibitor may be, but is not particularly limited to, any general route through which the peptide, the conjugate thereof, or the composition; and the SGLT-2 inhibitor may reach a target *in vivo*, for example, intraperitoneal administration, intravenous administration, intramuscular administration, subcutaneous administration, intradermal administration, oral administration, topical administration, intranasal administration, intrapulmonary administration, intrarectal administration, etc.

In the method of the present invention, the peptide or the conjugate thereof; and the SGLT-2 inhibitor may be administered through the same route of administration or through the different routes of administration, and the route of administration of the drugs to be administered in combination may be independent of each other.

Further, the peptide, the conjugate thereof, or the composition; and the SGLT-2 inhibitor may be formulated into a unit dosage form suitable for a patient's body according to common methods in the pharmaceutical field, and specifically, may be formulated into a preparation useful for the administration of a protein drug, and may be administered by an administration method commonly used in the art through an oral or parenteral route, such as through skin, intravenous, intramuscular, intraarterial, intramedullary, intrathecal, intraventricular, pulmonary, transdermal, subcutaneous, intraperitoneal, intranasal, intra-gastric, topical, sublingual, vaginal, or rectal route, but is not limited thereto.

Although not limited, the peptide of the present invention, the conjugate thereof, or the composition including the same may be administered once every 3 days, once a week, once every 2 weeks, once every 4 weeks, or once a month, but is not limited thereto.

Meanwhile, the frequency of administration of the SGLT-2 inhibitor is not limited as long as it may simultaneously exert the blood glucose lowering and weight loss effects by being administered in combination with the peptide of the present invention, the conjugate thereof, or the composition including the same. For example, the SGLT-2 inhibitor may be administered once every 3 days, once a week, once every 2 weeks, once every 4 weeks, or once a month. Further, the SGLT-2 inhibitor may be administered at the same interval as the peptide or the conjugate thereof, or at different intervals, for example, at intervals of 1 day, 2 days, 3 days, 4 days, 5 days, 6 days, 7 days, 8 days, 9 days, or 10 days or more, but is not limited to thereto.

The method of the present invention may include administering the composition including the peptide or the conjugate thereof; and the SGLT-2 inhibitor in a pharmaceutically effective amount. The appropriate total daily usage may be determined by a physician within the scope of sound medical judgment, and the composition may be administered once or in a few divided doses. However, with respect to the objects of the present invention, it is preferable that the specific therapeutically effective dose level for any particular patient may vary depending on a variety of factors, including the kind and degree of a reaction to be achieved, the specific composition, including whether or not any other agents is used in some cases, a patient's age, weight, general health conditions, sex, and diet, the time of administration, route of administration, and rate of the excretion of the composition, the duration of the treatment, and other drugs used in combination or coincidentally with the specific composition, and similar factors well known in the medical arts.

In the method of the present invention, the peptide or the long-acting conjugate thereof may be administered in combination with the SGLT-2 inhibitor at an appropriate ratio. For example, based on one dose, the peptide of the present invention or the long-acting conjugate thereof may be administered in an amount of 0.1 mg to 100 mg, 0.5 mg to 80 mg, 1 mg to 60 mg, 1 mg to 25 mg, 5 mg to 20 mg, or 10 mg to 15 mg, and the SGLT-2 inhibitor may be administered in an amount of 1 mg to 50 mg, 3 mg to 30 mg, or 5 mg to 25 mg, but are not limited thereto.

In accordance with still another aspect of the present invention, there is provided a method of preventing or treating diabetes and/or obesity, the method including the step of administering the combination or the pharmaceutical composition, and/or using the pharmaceutical kit to/in a subject in need thereof.

In accordance with still another aspect of the present invention, there is provided a method of preventing or treating metabolic syndrome, liver disease, lung disease, or respiratory infections, the method including the step of administering the combination or the pharmaceutical composition, and/or using the pharmaceutical kit to/in a subject in need thereof.

The combination, pharmaceutical composition, pharmaceutical kit, subject, administration, diabetes, obesity, metabolic syndrome, liver disease, lung disease, or respiratory infections, combination administration, preventing, and treating are as described above.

For specific example, the method may be a method of administering the peptide or the conjugate thereof in combination with the SGLT-2 inhibitor, but is not limited thereto.

In accordance with still another aspect of the present invention, there is provided use of the peptide, the conjugate thereof, or the composition including the same in preventing or treating diabetes and/or obesity, and/or use thereof in the preparation of a prophylactic or therapeutic agent for diabetes and/or obesity.

In accordance with still another aspect of the present invention, there is provided use of the peptide, the conjugate thereof, or the composition including the same in preventing or treating metabolic syndrome, liver disease, lung disease, or respiratory infections, and/or use thereof in the preparation of a prophylactic or therapeutic agent for metabolic syndrome, liver disease, lung disease, or respiratory infections.

The peptide, conjugate thereof, composition, diabetes, obesity, metabolic syndrome, liver disease, lung disease, or respiratory infections, preventing, and treating are as described above.

Specifically, the use may be use for administration of the peptide or the conjugate thereof in combination with the SGLT-2 inhibitor.

In accordance with still another aspect of the present invention, there is provided use of the combination, pharmaceutical composition, or pharmaceutical kit in preventing or treating diabetes and/or obesity, and/or use thereof in the preparation of a prophylactic or therapeutic agent for diabetes and/or obesity.

In accordance with still another aspect of the present invention, there is provided use of the combination, pharmaceutical composition, or pharmaceutical kit in preventing or treating metabolic syndrome, liver disease, lung disease, or respiratory infections, and/or use thereof in the preparation of a prophylactic or therapeutic agent for metabolic syndrome, liver disease, lung disease, or respiratory infections.

The combination, pharmaceutical composition, pharmaceutical kit, subject, administration, diabetes, obesity, metabolic syndrome, liver disease, lung disease, or respiratory infections, preventing, treating, combination administration, preventing, and treating are as described above.

Specifically, the use may be use for administration of the peptide or the conjugate thereof in combination with the SGLT-2 inhibitor.

Hereinafter, the present invention will be described in more detail with reference to the following exemplary embodiments. However, the following exemplary embodiments are only for illustrating the present invention, and the scope of the present invention is not intended to be limited thereby.

### Example 1: Preparation of triple agonists

Triple agonists exhibiting activities to all of GLP-1, GIP, and glucagon receptors were prepared, and sequences thereof are shown in Table 1 below.

**[Table 1]**

| **SEQ ID NO.** | **Sequence** | **Information** |
|---|---|---|
| 1 | H X Q G T F T S D V S S Y L D G Q A A K E F I A W L V K G C | |
| 2 | H X Q G T F T S D V S S Y L D G Q A Q K E F I A W L V K G C | |
| 3 | H X Q G T F T S D V S S Y L L G Q A A K Q F I A W L V K G G G P S S G A P PP S C | |
| 4 | H X Q G T F T S D V S S Y L L G Q Q Q K E F I A W L V K G C | |
| 5 | H X Q G T F T S D V S S Y L L G Q Q Q K E F I A W L V K G G G P S S G A P P P S C | |
| 6 | H X Q G T F T S D V S S Y L D G Q A A K E F V A W L L K G C | |
| 7 | H X Q G T F T S D V S K Y L D G Q A A K E F V A W L L K G C | |
| 8 | H X Q G T F T S D V S K Y L D G Q A A Q E F V A W L L K G C | |
| 9 | H X Q G T F T S D V S K Y L D G Q A A Q E F V A W L L A G C | |
| 10 | H X Q G T F T S D V S K Y L D G Q A A Q E F V A W L L A G G G P S S G A P P P S C | |
| 11 | CA G E G T F T S D L S K Y L D S R R Q Q L F V Q W L K A G G P S S G A P P P S H G | |
| 12 | CA G E G T F I S D L S K Y M D E Q A V Q L F V E W L M A G G P S S G A P P P S H G | |
| 13 | CA G E G T F I S D Y S I Q L D E I A V Q D F V E W L L A Q K P S S G A P P P S H G | |
| 14 | CA G Q G T F T S D Y S I Q L D E I A V R D F V E W L K N G G P S S G A P P P S H G | |
| 15 | CA G Q G T F T S D L S K Q M D E E A V R L F I E W L K N G G P S S G A P P P S H G | |
| 16 | CA G Q G T F T S D L S K Q M D S E A Q Q L F I E W L K N G G P S S G A P P P S H G | |
| 17 | CA G Q G T F T S D L S K Q M D E E R A R E F I E W L L A Q K P S S G A P P P S H G | |
| 18 | CA G Q G T F T S D L S K Q M D S E R A R E F I E W L K N T G P S S G A P P P S H G | |
| 19 | CA G Q G T F T S D L S I Q Y D S E H Q R D F I E W L K D T G P S S G A P P P S H G | |
| 20 | CA G Q G T F T S D L S I Q Y E E E A Q Q D F V E W L K D T G P S S G A P P P S H G | |
| 21 | Y X Q G T F T S D Y S K Y L D **E** C R A **K** E F V Q W L L D H H P S S G Q P PP S | Ring formation |
| 22 | Y X Q G T F T S D Y S K C L D **E** K R A **K** E F V Q W L L D H H P S S G Q P P P S | Ring formation |
| 23 | Y X Q G T F T S D Y S K Y L D **E** C R A **K** E F V Q W L LA Q KG K K N D W K H N I T | Ring formation |
| 24 | Y X Q G T F T S D Y S K Y L D **E** C R A **K** E F V Q W L K N G G P S S G A P P P S | Ring formation |
| 25 | H X Q G T F T S D C S K Y L D E R A A Q D F V Q W L L D G G P S S G A P P P S | |
| 26 | H X Q G T F T S D C S K Y L D S R A A Q D F V Q W L L D G G P S S G A P P P S | |
| 27 | H X Q G T F T S D Y S K Y L D E R A C Q D F V Q W L L D Q G G P S S G A P P P S | |
| 28 | H X Q G T F T S D Y S K Y L D E K R A Q E F V C W L L A Q K G K K N D W K H N I T | |
| 29 | H X Q G T F T S D Y S K Y L D **E** K A A **K** E F V Q W L L N T C | Ring formation |
| 30 | H X Q G T F T S D Y S K Y L D **E** K A Q **K** E F V Q W L L D T C | Ring formation |
| 31 | H X Q G T F T S D Y S K Y L D **E** K A C **K** E F V Q W L L A Q | Ring formation |
| 32 | H X Q G T F T S D Y S K Y L D **E** K A C **K** D F V Q W L L D G G P S S G A P P P S | Ring formation |
| 33 | H X Q G T F T S D Y S I A M D **E** I H Q **K** D F V N W L L A Q K C | Ring formation |
| 34 | H X Q G T F T S D Y S K Y L D **E** K R Q **K** E F V N W L L A Q K C | Ring formation |
| 35 | H X Q G T F T S D Y S I A M D **E** I H Q **K** D F V N W L L N T K C | Ring formation |
| 36 | H X Q G T F T S D Y S K Y L C **E** K R Q **K** E F V Q W L L N G G P S S G A P P P S G | Ring formation |
| 37 | H X Q G T F T S D Y S K Y L D **E** C R Q **K** E F V Q W L L N G G P S S G A P P P S G | Ring formation |
| 38 | CA X Q G T F T S D K S S Y L D E R A A Q D F V Q W L L D G G P S S G A P P P S S | |
| 39 | H X Q G T F T S D Y S K Y L D G Q H A Q C F V A W L L A G G G P S S G A P P P S | |
| 40 | H X Q G T F T S D K S K Y L D E R A C Q D F V Q W L L D G G P S S G A P P P S | |
| 41 | H X Q G T F T S D K S K Y L D E C A A Q D F V Q W L L D G G P S S G A P P P S | |
| 42 | Y X Q G T F T S D Y S K Y L D **E** K R A **K** E F V Q W L L D H H P S S G Q P P P S C | Ring formation |
| 43 | Y X Q G T F T S D Y S K Y L D **E** K R A **K** E F V Q W L L D H H C S S G Q P P P S | Ring formation |
| 44 | H G Q G T F T S D C S K Q L D G Q A A Q E F V A W L L A G G P S S G A P P P S | |
| 45 | H G Q G T F T S D C S K Y M D G Q A A Q D F V A W L L A G G P S S G A P P P S | |
| 46 | H G Q G T F T S D C S K Y L D E Q H A Q E F V A W L L A G G P S S G A P P P S | |
| 47 | H G Q G T F T S D C S K Y L D G Q R A Q E F V A W L L A G G P S S G A P P P S | |
| 48 | H G Q G T F T S D C S K Y L D G Q R A Q D F V N W L L A G G P S S G A P P P S | |
| 49 | CA X Q G T F T S D Y S I C M D **E** I H Q **K** D F V N W L L N T K | Ring formation |
| 50 | H X Q G T F T S D Y S K Y L D **E** K R A **K** E F V Q W L L D H H P S S G Q P P P S C | Ring formation |
| 51 | H X Q G T F T S D Y S K Y L D **E** K R Q B **K** E F V Q W L L N T C | Ring formation |
| 52 | H X Q G T F T S D Y S K Y L D **E** K R Q **K** E F V Q W L L D T C | Ring formation |
| 53 | H X E G T F T S D Y S I A M D **E** I H Q **K** D F V N W L L A Q C | Ring formation |
| 54 | H X E G T F T S D Y S I A M D **E** I H Q **K** D F V D W L L A E C | Ring formation |
| 55 | H X Q G T F T S D Y S I A M D **E** I H Q **K** D F V N W L L A Q C | Ring formation |
| 56 | H X Q G T F T S D Y S K Y L D **E** K R Q **K** E F V N W L L A Q C | Ring formation |
| 57 | H X Q G T F T S D Y S I A M D **E** I H Q **K** D F V N W L L N T C | Ring formation |
| 58 | H X Q G T F T S D Y S K Y L D **E** K R Q **K** E F V Q W L L N T K C | Ring formation |
| 59 | C A X Q G T F T S D Y S I C M D **E** K H Q **K** D F V N W L L N T K | Ring formation |
| 60 | C A X Q G T F T S D Y S I A M D **E** K H C **K** D F V N W L L N T K | Ring formation |
| 61 | C A X Q G T F T S D Y S I A M D **E** I A C **K** D F V N W L L N T K | Ring formation |
| 62 | C A X Q G T F T S D K S K Y L D E R A A Q D F V Q W L L D G G P S S G A P P P S | |
| 63 | C A X Q G T F T S D C S K Y L D E R A A Q D F V Q W L L D G G P S S G A P P P S | |
| 64 | Y X Q G T F T S D Y S K Y L D **E** C A A **K** E F V Q W L L D H H P S S G Q P P P S | Ring formation |
| 65 | H X Q G T F T S D Y S K C L D **E** K R A **K** E F V Q W L L D H H P S S G Q P P P S | Ring formation |
| 66 | Y X Q G T F T S D Y S K Y L D **E** C R A **K** D F V Q W L L D H H P S S G Q P P P S | Ring formation |
| 67 | Y X Q G T F T S D Y S K Y L D **E** C A A **K** D F V Q W L L D H H P S S G Q P P P S | Ring formation |
| 68 | Y X Q G T F T S D Y S K C L D **E** K A A **K** E F V Q W L L D H H P S S G Q P P P S | Ring formation |
| 69 | Y X Q G T F T S D Y S K C L D **E** R A A **K** E F V Q W L L D H H P S S G Q P P P S | Ring formation |
| 70 | Y X Q G T F T S D Y S K C L D **E** K R A **K** D F V Q W L L D H H P S S G Q P P P S | Ring formation |
| 71 | Y X Q G T F T S D Y S K Y L D **E** R A C **K** D F V Q W L L D H H P S S G Q P P P S | Ring formation |
| 72 | Y X Q G T F T S D C S K Y L D **E** R A A **K** D F V Q W L L D H H P S S G Q P P P S | Ring formation |
| 73 | C A X Q G T F T S D Y S K Y L D **E** C R A **K** E F V Q W L L D H H P S S G Q P P P S | Ring formation |
| 74 | C A X Q G T F T S D Y S K C L D **E** K R A **K** E F V Q W L L D H H P S S G Q P P P S | Ring formation |
| 75 | Y X Q G T F T S D Y S K Y L D **E** K A A **K** E F V Q W L L D H H P S S G Q P P P S C | Ring formation |
| 76 | Y X Q G T F T S D Y S K Y L D **E** K R A **K** D F V Q W L L D H H P S S G Q P P P S C | Ring formation |
| 77 | Y X Q G T F T S D Y S K Y L D **E** K A A **K** D F V Q W L L D H H P S S G Q P P P S C | Ring formation |
| 78 | H X Q G T F T S D Y S K Y L D **E** K R Q **K** E F V Q W L L D T K C | Ring formation |
| 79 | H X E G T F T S D Y S I A M D **E** I H Q **K** D F V N W L L A Q K C | Ring formation |
| 80 | H X E G T F T S D Y S I A M D **E** I H Q **K** D F V D W L L A E K C | Ring formation |
| 81 | CA X Q G T F T S D Y S K Y L D **E** K R Q **K** E F V Q W L L N T C | Ring formation |
| 82 | CA X Q G T F T S D Y S K Y L D **E** K R Q **K** E F V Q W L L D T C | Ring formation |
| 83 | CA X E G T F T S D Y S I A M D **E** I H Q **K** D F V N W L L A Q C | Ring formation |
| 84 | CA X E G T F T S D Y S I A M D **E** I H Q **K** D F V D W L L A E C | Ring formation |
| 85 | CA X Q G T F T S D Y S I A M D **E** I H Q **K** D F V N W L L A Q C | Ring formation |
| 86 | CA X Q G T F T S D Y S K Y L D **E** K R Q **K** E F V N W L L A Q C | Ring formation |
| 87 | CA X Q G T F T S D Y S I A M D **E** I H Q **K** D F V N W L L N T C | Ring formation |
| 88 | CA X Q G T F T S D Y S K Y L D **E** K R Q **K** E F V Q W L L N T K C | Ring formation |
| 89 | CA X Q G T F T S D Y S K Y L D **E** K R Q **K** E F V Q W L L D T K C | Ring formation |
| 90 | CA X E G T F T S D Y S I A M D **E** I H Q **K** D F V N W L L A Q K C | Ring formation |
| 91 | CA X E G T F T S D Y S I A M D **E** I H Q **K** D F V D W L L A E K C | Ring formation |
| 92 | CA X Q G T F T S D Y S I A M D **E** I H Q **K** D F V N W L L A Q K C | Ring formation |
| 93 | CA X Q G T F T S D Y S K Y L D **E** K R Q **K** E F V N W L L A Q K C | Ring formation |
| 94 | CA X Q G T F T S D Y S I A M D **E** I H Q **K** D F V N W L L N T K C | Ring formation |
| 95 | Y X Q G T F T S D Y S K Y L D **E** K R A **K** E F V Q W L L C H H P S S G Q P P P S | Ring formation |
| 96 | Y X Q G T F T S D Y S K Y L D **E** K R A **K** E F V Q W L L D H C P S S G Q P P P S | Ring formation |
| 97 | Y X Q G T F T S D Y S K Y L D **E** K R A **K** E F V Q W L L D C H P S S G Q P P P S | Ring formation |
| 98 | Y X Q G T F T S D Y S K A L D **E** K A A **K** E F V N W L L D H H P S S G Q P P P S C | Ring formation |
| 99 | Y X Q G T F T S D Y S K A L D **E** K A A **K** D F V N W L L D H H P S S G Q P P P S C | Ring formation |
| 100 | Y X Q G T F T S D Y S K A L D **E** K A A **K** E F V Q W L L D Q H P S S G Q P P P S C | Ring formation |
| 101 | Y X Q G T F T S D Y S K A L D **E** K A A **K** E F V N W L L D Q H P S S G Q P P P S C | Ring formation |
| 102 | Y X Q G T F T S D Y S K A L D **E** K A A **K** D F V N W L L D Q H P S S G Q P P P S C | Ring formation |

In the sequences shown in Table 1, the amino acid marked with X represents 2-aminoisobutyric acid (Aib) which is a non-native amino acid, and the underlined amino acids indicate the formation of a ring between the bold and underlined amino acids. Further, in Table 1, CA represents 4-imidazoacetyl, and Y represents tyrosine.

### Example 2: Preparation of long-acting conjugates of triple agonists

To PEGylate cysteine residues of the triple agonists (SEQ ID NOS: 21, 22, 42, 43, 50, 77, and 96) of Example 1 with 10 kDa of PEG having a maleimide group and an aldehyde group at the respective ends, i.e., maleimide-PEG-aldehyde (10 kDa, NOF, Japan), the triple agonists and the maleimide-PEG-aldehyde were reacted at a molar ratio of 1:1 to 3, a protein concentration of 1 mg/mL to 5 mg/mL, and a low temperature for 0.5 to 3 hours. The reaction was conducted under an environment in which 20-60% isopropanol was added to 50 mM Tris buffer (pH 7.5). Upon completion of the reaction, the reaction solutions were applied to SP Sepharose HP (GE Healthcare, USA) to purify triple agonists having mono-PEGylated cysteines.

Next, the purified mono-PEGylated triple agonists and an immunoglobulin Fc (the homodimer of SEQ ID NO: 123) were reacted at a molar ratio of 1:1 to 5, a protein concentration of 10 mg/mL to 50 mg/mL, and 4°C to 8°C for 12 to 18 hours. The reaction was conducted under an environment in which 10 mM to 50 mM sodium cyanoborohydride as a reducing agent and 10% to 30% isopropanol were added to a 100 mM potassium phosphate butter (pH 6.0). Upon completion of the reaction, the reaction solutions were applied to the butyl sepharose FF purification column (GE Healthcare, USA) and Source ISO purification column (GE Healthcare, USA) to purify conjugates including triple agonists and immunoglobulin Fc. The purified long-acting conjugates have a structure in which a triple agonist peptide, a polyethylene glycol (PEG) linker, and an Fc dimer are covalently linked at a molar ratio of 1:1:1 in the molecule, wherein the PEG linker is linked to only one of the two polypeptide chains of the Fc dimer.

Meanwhile, in the immunoglobulin Fc, two monomers having the amino acid sequence of SEQ ID NO: 123 (consisting of 221 amino acids) form a homodimer through a disulfide bond between cysteines, which correspond to the amino acid at position 3 of each monomer, wherein the monomers of the homodimer, each independently, form an intra-disulfide bond between the cysteines at positions 35 and 95 and an intra-disulfide bond between the cysteines at positions 141 and 199, respectively.

The purity that was analyzed by reverse-phase chromatography, size-exclusion chromatography, and ion-exchange chromatography after the preparation was 95% or higher.

Here, the conjugate in which the triple agonist of SEQ ID NO: 21 and the immunoglobulin Fc were linked via the PEG linker was named 'the conjugate including SEQ ID NO: 21 and immunoglobulin Fc' or 'the long-acting conjugate of SEQ ID NO: 21', and these may be used interchangeably herein.

Here, the conjugate in which the triple agonist of SEQ ID NO: 22 and the immunoglobulin Fc were linked via the PEG linker was named 'the conjugate including SEQ ID NO: 22 and immunoglobulin Fc' or 'the long-acting conjugate of SEQ ID NO: 22', and these may be used interchangeably herein.

Here, the conjugate in which the triple agonist of SEQ ID NO: 42 and the immunoglobulin Fc were linked via the PEG linker was named 'the conjugate including SEQ ID NO: 42 and immunoglobulin Fc' or 'the long-acting conjugate of SEQ ID NO: 42', and these may be used interchangeably herein.

Here, the conjugate in which the triple agonist of SEQ ID NO: 43 and the immunoglobulin Fc were linked via the PEG linker was named 'the conjugate including SEQ ID NO: 43 and immunoglobulin Fc' or 'the long-acting conjugate of SEQ ID NO: 43', and these may be used interchangeably herein.

Here, the conjugate in which the triple agonist of SEQ ID NO: 50 and the immunoglobulin Fc were linked via the PEG linker was named 'the conjugate including SEQ ID NO: 50 and immunoglobulin Fc' or 'the long-acting conjugate of SEQ ID NO: 50', and these may be used interchangeably herein.

Here, the conjugate in which the triple agonist of SEQ ID NO: 77 and the immunoglobulin Fc were linked via the PEG linker was named 'the conjugate including SEQ ID NO: 77 and immunoglobulin Fc' or 'the long-acting conjugate of SEQ ID NO: 77', and these may be used interchangeably herein.

Here, the conjugate in which the triple agonist of SEQ ID NO: 96 and the immunoglobulin Fc were linked via the PEG linker was named 'the conjugate including SEQ ID NO: 96 and immunoglobulin Fc' or 'the long-acting conjugate of SEQ ID NO: 96', and these may be used interchangeably herein.

### Experimental Example 1: Measurement of in vitro activities of triple agonists and long-acting conjugates thereof

To determine the activities of the triple agonists and the long-acting conjugates thereof prepared in Examples 1 and 2, the cell activity was measured *in vitro* by using cell lines in which GLP-1 receptor, glucagon (GCG) receptor, and GIP receptor were transformed, respectively.

The cell lines were obtained by transforming Chinese hamster ovary (CHO) cells to express human GLP-1 receptor, human GCG receptor, and human GIP receptor, respectively, and are suitable for the measurement of the activities of GLP-1, GCG, and GIP. Therefore, the activities for the respective parts were measured using the transformed cell lines, respectively.

For the measurement of the GLP-1 activity of the triple agonists and the long-acting conjugates thereof prepared in Examples 1 and 2, human GLP-1 was subjected to a 4-fold serial dilution from 50 nM to 0.000048 nM, and the triple agonists and long-acting conjugates thereof prepared in Examples 1 and 2 were subjected to a 4-fold serial dilution from 400 nM to 0.00038 nM. The culture medium was removed from the cultured CHO cells expressing the human GLP-1 receptor, and 5 µl of each of the serially diluted materials was added to the cells, and then 5 µl of a buffer containing cAMP antibody was added, followed by incubation at room temperature for 15 minutes. Then, 10 µl of a detection mix containing a cell lysis buffer was added to lyse the cells, followed by incubation at room temperature for 90 minutes. The cell lysate, in which the reaction was completed, was applied to a LANCE cAMP kit (PerkinElmer, USA) to calculate EC₅₀ values through accumulated cAMP, and then the values were compared with each other. The relative titers compared to human GLP-1 are shown in Tables 2 and 3 below.

For the measurement of the GCG activity of the triple agonists and the long-acting conjugates thereof prepared in Examples 1 and 2, human GCG was subjected to a 4-fold serial dilution from 50 nM to 0.000048 nM, and the triple agonists and long-acting conjugates thereof prepared in Examples 1 and 2 were subjected to a 4-fold serial dilution from 400 nM to 0.00038 nM. The culture medium was removed from the cultured CHO cells expressing the human GCG receptor, and 5 µl of each of the serially diluted materials was added to the cells, and then 5 µl of a buffer containing cAMP antibody was added, followed by incubation at room temperature for 15 minutes. Then, 10 µl of a detection mix containing a cell lysis buffer was added to lyse the cells, followed by incubation at room temperature for 90 minutes. The cell lysate, in which the reaction was completed, was applied to a LANCE cAMP kit (PerkinElmer, USA) to calculate EC₅₀ values through accumulated cAMP, and then the values were compared with each other. The relative titers com-pared to human GCG are shown in Tables 2 and 3 below.

For the measurement of the GIP activity of the triple agonists and the long-acting conjugates thereof prepared in Examples 1 and 2, human GIP was subjected to a 4-fold serial dilution from 50 nM to 0.000048 nM, and the triple agonists and long-acting conjugates thereof prepared in Examples 1 and 2 were subjected to a 4-fold serial dilution from 400 nM to 0.00038 nM. The culture medium was removed from the cultured CHO cells expressing the human GIP receptor, and 5 µl of each of the serially diluted materials was added to the cells, and then 5 µl of a buffer containing cAMP antibody was added, followed by incubation at room temperature for 15 minutes. Then, 10 µl of a detection mix containing a cell lysis buffer was added to lyse the cells, followed by incubation at room temperature for 90 minutes. The cell lysate, in which the reaction was completed,, was applied to a LANCE cAMP kit (PerkinElmer, USA) to calculate EC₅₀ values through accumulated cAMP, and then the values were compared with each other. The relative titers compared to human GIP are shown in Tables 2 and 3 below.

**[Table 2]**

| Relative titer ratio of triple agonists | | | |
|---|---|---|---|
| | ***In vitro* activity compared with native peptide (%)** | | |
| **SEQ ID NO.** | vs GLP-1 | vs Glucagon | vs GIP |
| 1 | 3.2 | <0.1 | <0.1 |
| 2 | 5.9 | <0.1 | <0.1 |
| 3 | 1.8 | <0.1 | <0.1 |
| 4 | 8.5 | <0.1 | <0.1 |
| 5 | 42.1 | <0.1 | <0.1 |
| 6 | 17.0 | <0.1 | <0.1 |
| 7 | 13.7 | <0.1 | <0.1 |
| 8 | 14.2 | 0.10 | <0.1 |
| 9 | 32.1 | 0.13 | <0.1 |
| 10 | 46.0 | <0.1 | <0.1 |
| 11 | 1.4 | <0.1 | <0.1 |
| 12 | 0.4 | <0.1 | <0.1 |
| 13 | < 0.1 | < 0.1 | < 0.1 |
| 14 | 28.0 | < 0.1 | < 0.1 |
| 15 | 79.2 | <0.1 | <0.1 |
| 16 | 2.1 | < 0.1 | < 0.1 |
| 17 | 0.2 | < 0.1 | < 0.1 |
| 18 | <0.1 | <0.1 | <0.1 |
| 19 | <0.1 | <0.1 | <0.1 |
| 20 | <0.1 | <0.1 | <0.1 |
| 21 | 17.8 | 267 | 22.7 |
| 22 | 20.1 | 140 | 59.7 |
| 23 | 4.01 | 9.3 | <0.1 |
| 24 | 41.2 | 9.3 | < 0.1 |
| 25 | 82.6 | 0.1 | <0.1 |
| 26 | 64.5 | 0.2 | <0.1 |
| 27 | 83.1 | 0.8 | 0.9 |
| 28 | 17.2 | 1.6 | <0.1 |
| 29 | 38.5 | 6.0 | <0.1 |
| 30 | 142 | 0.7 | 0.8 |
| 31 | 135 | 2.2 | 2.4 |
| 32 | 151 | 1.7 | 8.8 |
| 33 | 24.5 | <0.1 | 10.4 |
| 34 | 19.1 | 0.92 | 0.6 |
| 35 | 7.5 | <0.1 | 1.3 |
| 36 | 37.4 | 0.39 | 0.2 |
| 37 | 236 | 6.21 | 2.2 |
| 38 | 2.3 | - | - |
| 39 | 13.9 | 0.53 | <0.1 |
| 40 | 75.2 | <0.1 | <0.1 |
| 41 | 34.3 | <0.1 | <0.1 |
| 42 | 33.9 | 205.8 | 7.8 |
| 43 | 12.6 | 88.4 | 3.70 |
| 44 | 1.3 | <0.1 | <0.1 |
| 45 | 6.6 | < 0.1 | < 0.1 |
| 46 | 1.4 | < 0.1 | < 0.1 |
| 47 | 2.4 | < 0.1 | < 0.1 |
| 48 | 1.5 | < 0.1 | < 0.1 |
| 49 | 29.8 | <0.1 | 3.3 |
| 50 | 67.4 | 50.5 | 2.7 |
| 51 | 14.4 | 2.0 | 0.1 |
| 52 | 44.1 | 7.5 | 0.3 |
| 53 | 161 | 8.4 | 1.3 |
| 54 | 30.6 | 1.4 | 0.1 |
| 55 | 27.1 | 0.7 | 2.4 |
| 56 | 57.9 | 4.9 | 0.8 |
| 57 | 11.7 | <0.1 | 0.3 |
| 58 | 39.1 | 2.6 | 0.2 |
| 59 | 40.3 | <0.1 | 4.0 |
| 60 | 106.2 | <0.1 | 8.2 |
| 61 | 59.8 | <0.1 | 2.8 |
| 62 | 5.2 | <0.1 | <0.1 |
| 63 | 15.3 | <0.1 | <0.1 |
| 64 | 64.6 | 60.1 | 92.9 |
| 65 | 95.4 | 25.2 | 11.6 |
| 66 | 15.8 | 172 | 17.2 |
| 67 | 28.5 | 46.2 | 39.8 |
| 68 | 27.9 | 8.8 | 107 |
| 69 | 24.3 | 9.6 | 62.8 |
| 70 | 15.1 | 71.3 | 64.4 |
| 71 | 90.1 | 12.7 | 94.7 |
| 72 | 11.5 | 1.0 | 1.6 |
| 73 | 22.6 | 5.4 | 3.0 |
| 74 | 12.9 | 0.9 | 1.0 |
| 75 | 35.1 | 8.5 | 18.0 |
| 76 | 10.3 | 47.6 | 11.7 |
| 77 | 38.7 | 12.2 | 35.5 |
| 78 | 51.0 | 14.0 | 0.12 |
| 79 | 41.5 | 4.9 | 1.4 |
| 80 | 8.1 | 0.0 | 0.1 |
| 81 | 7.8 | 0.3 | <0.1 |
| 82 | 9.5 | 1.1 | <0.1 |
| 83 | 47.3 | 1.3 | 0.4 |
| 84 | 4.2 | <0.1 | <0.1 |
| 85 | 4.3 | <0.1 | 0.3 |
| 86 | 28.4 | 0.4 | 0.2 |
| 87 | 0.9 | <0.1 | <0.1 |
| 88 | 9.6 | 0.3 | <0.1 |
| 89 | 7.1 | 0.7 | <0.1 |
| 90 | 7.4 | <0.1 | <0.1 |
| 91 | 31.9 | 16.8 | 0.3 |
| 92 | 0.8 | <0.1 | 0.4 |
| 93 | 5.7 | 0.3 | 0.7 |
| 94 | 0.5 | <0.1 | <0.1 |
| 95 | 2.1 | 0.4 | <0.1 |
| 96 | 34.4 | 194.8 | 5.2 |
| 97 | 10.5 | 62.8 | 2.6 |
| 98 | 28.1 | 8.2 | 47.1 |
| 99 | 20.9 | 14.9 | 57.7 |
| 100 | 42.2 | 12.7 | 118.5 |
| 101 | 23.2 | 13.9 | 40.1 |
| 102 | 23.3 | 29.5 | 58.0 |

**[Table 3]**

| Relative titer ratio of long-acting conjugates of triple agonists | | | |
|---|---|---|---|
| Long-acting conjugate | *In vitro* activity compared with native peptide (%) | | |
| | vs GLP-1 | vs Glucagon | vs GIP |
| 21 | 0.1 | 1.6 | 0.2 |
| 22 | 0.1 | 0.9 | 0.5 |
| 42 | 3.1 | 23.1 | 1.2 |
| 43 | 2.1 | 13.5 | 0.6 |
| 50 | 15.4 | 6.9 | 0.7 |
| 77 | 6.7 | 1.7 | 6.6 |
| 96 | 0.3 | 4.0 | 0.3 |

The triple agonists and the conjugates thereof have a function as a triple agonist capable of activating all of GLP-1 receptor, GIP receptor, and glucagon receptor.

### Experimental Example 2: Confirmation of blood glucose lowering and weight loss effects of combination administration of long-acting conjugate and SGLT-2 inhibitor in DIO/STZ rats

To confirm the blood glucose lowering effect of combination administration of the long-acting conjugate of SEQ ID NO: 42 prepared in Examples 1 and 2 and an SGLT-2 inhibitor, the long-acting conjugate of SEQ ID NO: 42 was administered for 4 weeks, in combination with empagliflozin which is an SGLT-2 inhibitor, to DIO/STZ rats which are known as obesity and diabetes models.

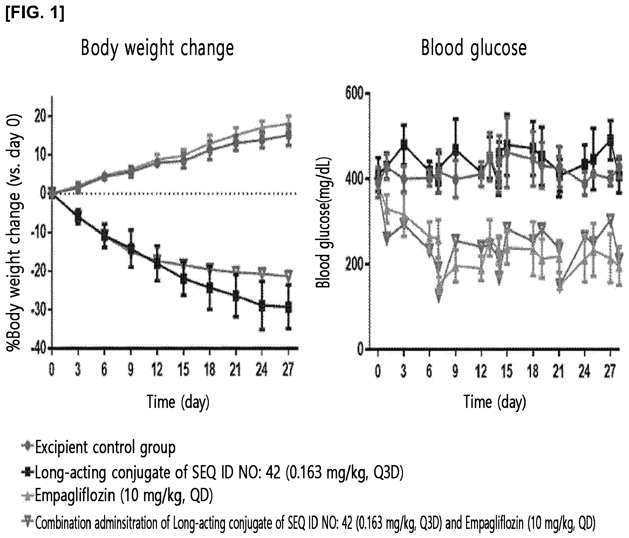

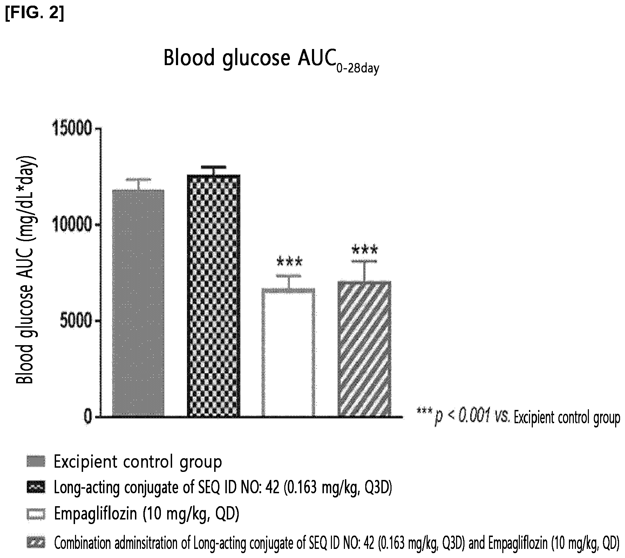

To confirm the blood glucose lowering effect according to the combination administration, the experimental groups were divided into an excipient control group, a group administered with only the long-acting conjugate of SEQ ID NO: 42 (0.163 mg/kg, Q3D), a group administered with only empagliflozin (10 mg/kg, QD), and a group administered with the long-acting conjugate of SEQ ID NO: 42 (0.163 mg/kg, Q3D) in combination with empagliflozin (10 mg/kg, QD), and the corresponding substances were subcutaneously administered repeatedly for 4 weeks. During repeated administration for 4 weeks, blood was taken from each rat's tail vein along with changes in body weight, and the degree of change in blood glucose was measured using a blood glucose meter (OneTouch^{®} Ultra^{®}). In the group administered with the long-acting conjugate of SEQ ID NO: 42 in combination with empagliflozin, the body weight and blood glucose levels were found to be effectively reduced (FIGS. 1 and 2).

Statistical analysis was performed using one-way ANOVA to compare between the excipient group (control group) and the experimental group.

These results indicate that combination administration of the long-acting triple agonist conjugate of the present invention with an SGLT-2 inhibitor such as empagliflozin exhibits the blood glucose lowering and weight loss effects at the same time, and therefore, therapeutic effects may be expected in patients with diabetes and/or obesity.

Based on the above description, it will be understood by those skilled in the art to which the present invention pertains that the present invention may be embodied in other specific forms without changing the technical spirit or essential characteristics thereof. In this regard, it should be understood that the above embodiments are not limitative, but illustrative in all aspects. The scope of the present invention should be construed as the meaning and scope of the appended claims rather than the detailed description, and all changes or variations derived from the equivalent concepts fall within the scope of the present invention.

## Claims

1. A pharmaceutical composition for preventing or treating metabolic syndrome, liver disease lung disease, or respiratory infections, the pharmaceutical composition comprising:
a pharmaceutically effective amount of a peptide including any one amino acid sequence of SEQ ID NOS: 1 to 102; and
a pharmaceutically acceptable excipient,
wherein the pharmaceutical composition is used in combination with a sodium-glucose cotransporter 2 (SGLT-2) inhibitor.

2. The pharmaceutical composition of claim 1, wherein the peptide is in the form of a long-acting conjugate, which is represented by Formula 1 below:
[Formula 1] X-L-F
wherein X represents a peptide including an amino acid sequence of any one of SEQ ID NOS: 1 to 102;
L represents a linker containing ethylene glycol repeating units;
F represents an immunoglobulin Fc region; and
- represents covalent linkages between X and L and between L and F, respectively.

3. The pharmaceutical composition of claim 1 or 2, wherein the metabolic syndrome is any one or more selected from the group consisting of diabetes, obesity, hyperlipidemia, and dyslipidemia.

4. The pharmaceutical composition of claim 1 or 2, wherein the SGLT-2 inhibitor is any one or more selected from the group consisting of empagliflozin, dapagliflozin, canagliflozin, remogliflozin, remogliflozin etabonate, sergliflozin, ipragliflozin, tofogliflozin, luseogliflozin, sotagliflozin, bexagliflozin, atigliflozin, and ertugliflozin.

5. The pharmaceutical composition of claim 1 or 2, wherein the composition has weight loss and/or blood glucose lowering effects.

6. The pharmaceutical composition of claim 1 or 2, wherein the composition is administered to diabetic patients with overweight or obesity.

7. The pharmaceutical composition of claim 1 or 2, wherein the peptide includes any one amino acid sequence of SEQ ID NOS: 21, 22, 42, 43, 50, 77, and 96.

8. The pharmaceutical composition of claim 1 or 2, wherein the peptide is C-terminally amidated.

9. The pharmaceutical composition of claim 1 or 2, wherein the peptide has a ring formed between amino acid residues.

10. The pharmaceutical composition of claim 2, wherein the immunoglobulin Fc region is aglycosylated.

11. The pharmaceutical composition of claim 2, wherein the immunoglobulin Fc region is an IgG4 Fc region.

12. The pharmaceutical composition of claim 2, wherein the immunoglobulin Fc region is a dimer consisting of two polypeptide chains, wherein one end of L is linked to only one polypeptide chain of the two polypeptide chains.

13. The pharmaceutical composition of claim 2, wherein in the conjugate, L is linked to F and X by covalent linkages formed by reacting one end of L with an amine group or thiol group of F and reacting the other end of L with an amine group or thiol group of X, respectively.

14. The pharmaceutical composition of claim 2, wherein L is polyethylene glycol.

15. The pharmaceutical composition of claim 2, wherein a formula weight of a moiety of the ethylene glycol repeating units in L is in the range of 1 kDa to 100 kDa.
